# EUROPEAN PATENT SPECIFICATION

(11) **EP 3 112 472 B1**
(45) Date of publication and mention of the grant of the patent: **27.03.2019**
(21) Application number: 15717522.5
(22) Date of filing: 27.02.2015
(51) Int. Cl.: C12P 33/00, C12N 9/02, C12R 1/34

(54) **SELECTIVE MYCOBACTERIUM SMEGMATIS MC2 155 RECOMBINANT MUTANTS AND USE THEREOF FOR PRODUCING 1,4-ANDROSTADIENE-3,17-DIONE OR 4-ANDROSTENE-3,17-DIONE FROM NATURAL STEROLS**
SELEKTIVE REKOMBINANTE MUTANTEN AUS MYCOBACTERIUM SMEGMATIS MC2 155 UND VERWENDUNG DAVON ZUR HERSTELLUNG VON 1,4-ANDROSTADIEN-3,17-DION ODER 4-ANDROSTEN-3,17-DION AUS NATÜRLICHEN STEROLEN
MUTANTS RECOMBINANTS SÉLECTIFS DE MYCOBACTERIUM SMEGMATIS MC2155 ET LEUR UTILISATION POUR LA PRODUCTION DE 1,4-ANDROSTADIÈNE-3,17-DIONE OU 4-ANDROSTÈNE-3,17-DIONE À PARTIR DE STÉROLS NATURELS

(30) Priority: 27.02.2014 ES 201430272
(43) Date of publication of application: 04.01.2017
(73) Proprietor: Consejo Superior De Investigaciones Científicas, 28006 Madrid (ES)
(72) Inventor: GARCÍA LÓPEZ, José Luis, 28006 Madrid (ES); UHÍA CASTRO, Iria, 28006 Madrid (ES); GALÁN SICILIA, Beatriz, 28006 Madrid (ES)
(74) Representative: Hoffmann Eitle
(86) International application number: PCT/ES2015/070144
(87) International publication number: WO 2015/128534

(56) References cited:
- WO-A1-2009/064924
- CN-A- 101 565 709
- US-A- 3 684 657
- MARINA V DONOVA ET AL: "Microbial steroid transformations: current state and prospects", APPLIED MICROBIOLOGY AND BIOTECHNOLOGY, vol. 94, no. 6, 6 May 2012 (2012-05-06), pages 1423-1447, XP035060657, ISSN: 1432-0614, DOI: 10.1007/S00253-012-4078-0 cited in the application
- GARCIA J L ET AL: "Catabolism and biotechnological applications of cholesterol degrading bacteria", MICROBIAL BIOTECHNOLOGY, vol. 5, no. 6, 1 November 2012 (2012-11-01), pages 679-699, XP002738411, ISSN: 0964-7562, DOI: 10.1111/J.1751-7915.2012.00331.X cited in the application
- A. ANDOR ET AL: "Generation of Useful Insertionally Blocked Sterol Degradation Pathway Mutants of Fast-Growing Mycobacteria and Cloning, Characterization, and Expression of the Terminal Oxygenase of the 3-Ketosteroid 9 -Hydroxylase in Mycobacterium smegmatis mc2155", APPLIED AND ENVIRONMENTAL MICROBIOLOGY, vol. 72, no. 10, 1 October 2006 (2006-10-01), pages 6554-6559, XP055195713, ISSN: 0099-2240, DOI: 10.1128/AEM.00941-06 cited in the application
- BRZOSTEK ANNA ET AL: "Identification and targeted disruption of the gene encoding the main 3-ketosteroid dehydrogenase in Mycobacterium smegmatis", MICROBIOLOGY, vol. 151, no. 7, 1 July 2005 (2005-07-01), pages 2393-2402, XP009141915, ISSN: 1350-0872 cited in the application
- WEI WEI ET AL: "Inactivation and Augmentation of the Primary 3-Ketosteroid-Delta(1)-Dehydrogenase in Mycobacterium neoaurum NwIB-01: Biotransformation of Soybean Phytosterols to 4-Androstene-3,17-Dione or 1,4-Androstadiene-3,17-Dione", APPLIED AND ENVIRONMENTAL MICROBIOLOGY, vol. 76, no. 13, 1 July 2010 (2010-07-01), pages 4578-4582, XP009141921, ISSN: 0099-2240, DOI: 10.1128/AEM.00448-10 cited in the application
- MARSHECK W J ET AL: "MICROBIAL DEGRADATION OF STEROLS", APPLIED MICROBIOLOGY, vol. 23, no. 1, 1 January 1972 (1972-01-01), pages 72-77, XP001154457, ISSN: 0003-6919 cited in the application
- FLEISCHMANN R D ET AL: "Mycobacterium smegmatis str. MC2 155, complete genome", EMBASE [ONLINE], 13 December 2006 (2006-12-13), XP002460864,

### SEQUENCE LISTING

<110> CONSEJO SUPERIOR DE INVESTIGACIONES CIENTIFICAS
<120> MUTANTES RECOMBINANTES SELECTIVOS DE MYCOBACTERIUM SMEGMATIS mc2155 Y SU USO PARA LA PRODUCCIÓN DE 1,4-ANDROSTADIEN-3,17-DIONA O 4-ANDROSTEN-3,17-DIONA A PARTIR DE ESTEROLES NATURALES
<130> 900712
<160> 10
<170> PatentIn version 3.5
<210> 1
   <211> 1035
   <212> DNA
   <213> Mycobacterium smegmatis
<400> 1
   gtgctcgaac tgcaggtggc cgaggtcgtc gaggagacct ccgatgcccg ctccctggtg      60
   ttcacggtgc ccgagggcgc cgagatcgcc gcggaccggc ttcggtactc ccccggccag     120
   ttcctgacgc tgcgggtccc cagcgaccgc accgggtcgg tcgcacgctg ctactcgctg     180
   tcctcgtcgc cgaccaccga cgaccggctc acggtgacgg tcaagcgcac cgccgacggt     240
   tacgcctcga actggctgtg cgacaacgcc cacgccggca tgcgcatcca cgtcctggcg     300
   ccctcgggga cgttcgtccc gaaggacctc gacaccgact tcctgctgct ggccgcgggc     360
   agcggcatca ccccgatgat ggcgatctgc aaatccgcgc tcgccgaggg caccggcaac     420
   gtggtgctga tctacgccaa ccgcgacgag aactcggtga tcttcgctgg cgcactgcgc     480
   gaactggccg cgaaataccc cgaccggctg accgtggtgc actggctgga gaccgtgcag     540
   ggcctgccga cggcggccgg cctcggcgcg ctcgccaagc cgttcgccgg gcgtgaggcc     600
   ttcatctgcg ggcccggtcc gttcatgacc gcagccgagg acgcgctgcg cgccgcgggc     660
   acccccgacg accacatcca catcgaggtg ttcaagtcgc tggagtccga tccgttcgcc     720
   gcggtcgtga tccccgagga cgacggggac gaccagggcc cggcgaccgc tgtggtgacg     780
   ctggacggca ccacacacga gatccgttgg ccgcgttcgg ccaagctgct cgatgtactg     840
   ctggacaagg gtctcgacgc gccgttctcg tgccgcgagg gacactgcgg cgcctgcgcg     900
   gtgctcaaga aatccggcga ggtgcacatg gagatcaacg acgttctgga gccgtcggat     960
   ctcgaggagg gtctgatcct gggttgccag gcgacacccg tgtccgattc cgtcgaagtc    1020
   acctacgacg aatag                                                     1035
<210> 2
   <211> 1701
   <212> DNA
   <213> Mycobacterium smegmatis
<400> 2
   atgactggac aggagtacga cgttgtggtg gtcgggagcg gtgctgccgg catggtcgcc      60
   gccctcaccg cagcccacca gggcctctcg acagtagtcg tagaaaaggc tccgcactac     120
   ggtggttcca ctgcgcggtc aggtggtggt gtgtggatcc cgaacaacga gatcctcaag     180
   cgcgacggcg tcaaggacac tcctgacgag gcgcggaaat acctgcacgc gatcatcggt     240
   gacgtcgtcc cggccgagaa gatcgacacg tatctggacc gcgggccgga aatgctgtcg     300
   ttcgtcctca agcactcgcc gctgaagctg tgctgggtgc ccggatactc cgactactac     360
   cccgagaccc cgggtggcaa gcccacgggg cggtcggtcg agccgaagcc gttcgacgcc     420
   aacaagctgg gccccgacct caaaggtctc gaaccgccgt acggcaaggt ccccatgaac     480
   atggtggtca tgcagcagga ctacgtgcgg ctcaaccagc tcaaacgtca tccgcgcggc     540
   gtactgcgca gcctcaaggt cggcatccgc gcgacgtggg gcaaggtcag cggcaagaac     600
   ctcgtcggta tgggccgcgc gctcatcgcg ccgctgcgta tcggcctgcg cgaggccggg     660
   gttccggtgc tgctgaacac cgcgctgacc gatctgtacg tcgaggacgg ccgcgtgctg     720
   ggcatctacg tccgcgacac gacggccggc gacgacgccg agccccggct gatccgcgca     780
   cgccacggcg tgatcctcgg atccggcggg ttcgagcaca acgagcagat gcgcgtgaag     840
   taccagcgcg cgcccatcac cacggagtgg accgtcggcg cggtcgccaa caccggcgac     900
   ggtatcgtcg ccgccgaaaa gctcggggcg gcactcgaac tcatggaaga cgcgtggtgg     960
   ggccccacgg ttccgctggt cggcgccccg tggttcgcct tgagcgagcg caactccccc    1020
   ggatcgatca tcgtcaacat gtccggcaag cgcttcatga acgaatccat gccctacgtc    1080
   gaggcatgcc accacatgta cggcggccag tacggccagg gccccggtcc cggcgagaac    1140
   atcccggcgt ggctcatctt cgaccagcaa taccgcgacc gctacatctt cgccggtttg    1200
   caaccagggc agcggatccc gtcgaagtgg ctggaatccg gtgtgatcgt caaggccgac    1260
   acgctggtcg aactggccga gaagaccggc ctgcccgccg atcagttcac ctcgaccatc    1320
   gagcgcttca acgggttcgc acgctcgggt gtcgacgagg acttccaccg cggcgagagt    1380
   gcctacgacc gttactacgg cgatcccacc aacaaaccca acccgaacct gggcgagatc    1440
   aagcacggcc ccttctacgc cgccaagatg gtgcccggtg acctcggcac caagggcggc    1500
   atccgcaccg acgtgcgcgg acgcgctctg cgcgacgacg attcggtgat cgagggtctg    1560
   tatgctgccg gcaatgtcag ctcaccggtg atgggccaca cctatccggg acccggcggc    1620
   accatcggtc ccgccatgac attcggttac ctggccgcac tcgacatcgc cgctaccgcg    1680
   gcggcctccc ggaagggctg a                                              1701
<210> 3
   <211> 29
   <212> DNA
   <213> Artificial Sequence
<220>
   <223> MSMEG_6039 Up F
<400> 3
   ctagctcgag ccagttgtgc acaccgatg 29
<210> 4
   <211> 28
   <212> DNA
   <213> Artificial Sequence
<220>
   <223> MSMEG_6039 Up R
<400> 4
   ctagactagt gcagcgtcag gaactggc 28
<210> 5
   <211> 30
   <212> DNA
   <213> Artificial Sequence
<220>
   <223> MSMEG_6039 Down F
<400> 5
   ctagactagt ggtgcacatg gagatcaacg 30
<210> 6
   <211> 29
   <212> DNA
   <213> Artificial Sequence
<220>
   <223> MSMEG_6039 Down R
<400> 6
   ctagtctaga gtaccagtcg atcggtgtc 29
<210> 7
   <211> 31
   <212> DNA
   <213> Artificial Sequence
<220>
   <223> MSMEG_5941 Up F
<400> 7
   ctagactagt gatgttgcga atgtcgatgt c 31
<210> 8
   <211> 30
   <212> DNA
   <213> Artificial Sequence
<220>
   <223> MSMEG_5941 Up R
<400> 8
   ctagtctaga ccaccacaac gtcgtactcc 30
<210> 9
   <211> 31
   <212> DNA
   <213> Artificial Sequence
<220>
   <223> MSMEG_5941 Down F
<400> 9
   ctagtctaga ccatgacatt cggttacctg g 31
<210> 10
   <211> 30
   <212> DNA
   <213> Artificial Sequence
<220>
   <223> MSMEG_5941 Down R
<400> 10
   ctaggagctc gcagggagat ctcgaaatcg 30

## Description

### TECHNICAL FIELD

The present invention is encompassed within the area of biotechnology in the chemical industry, pharmaceutical industry and food industry fields, among others.

The developed product makes it applicable for producing steroids by means of bacterial biotransformations, but particularly for producing steroids from natural steroids.

### PRIOR ART

### Steroids

Steroids are terpenoid lipids with a specific structure containing a cyclopentanoperhydrophenanthrene or gonane nucleus with four fused rings (A-D); this basic structure is modified by adding various functional groups such as carbonyls and hydroxyls (hydrophilic) or hydrocarbon chains (hydrophobic). The physiological activity of steroids depends on their structure, i.e., the type, number and position of the functional groups bound to the steroid nucleus, on their configurational and structural isomerism, i.e., stereoisomerism and regioisomerism, and on the oxidation state of the rings.

Steroids are widespread in nature and present in all types of organisms. Hundreds of sterols and related compounds produced in plants, such as phytosterols, for example; in insects, such as ecdysteroids, for example; in vertebrates, such as cholesterol, corticosteroids or steroid hormones, for example; and in lower eukaryotes (yeasts and fungi), such as ergosterol, for example, have been identified.

Steroid-type pharmaceutical products are very important today for maintaining quality of life because many steroids are used as antitumor, anti-inflammatory, antimicrobial, antiviral, antifungal, antiestrogenic, anticonvulsant and antiallergic agents. Furthermore, others are used as agents for the prevention and therapy of many other diseases, such as hormone-dependent breast and prostate cancers, certain forms of colon cancer, obesity, diabetes, rheumatoid arthritis, hypertension, asthma, eczema, inflammations, metabolic disorders, neurodegenerative diseases in the elderly, or central nervous system diseases, among many others. Androgens, anabolic steroids, estrogens, and corticosteroids, among others, are included herein.

About 300 steroid drugs have been approved up until now for clinical use and this number tends to grow. Steroid medicinal products are among the medical products most widely available on the market and represent the second major category of drugs together with antibiotics. Annual production of steroids is estimated to be more than one million tons with a market worth billions of Euros.

Many steroids used as drugs are chemically synthesized, but it has been known for a long time that microbial transformation of steroids is a powerful tool for generating new steroid drugs, as well as for efficiently producing intermediate products key for the chemical synthesis of such drugs. Bioconversions allow modifying steroids in positions of the molecule hardly available to or accessible for chemical agents, and functionalization of the molecule can be done regiospecifically and stereospecifically. Furthermore, several reactions can be completed in a single step by means of bioconversion. The foregoing and other advantages of biotransformations have entailed the extensive expansion of microbial technologies in the field of steroids, such that applications of microorganisms for modifying steroids have been reviewed in a number of articles over the past few years (for example, Marina V. Donova et al., Microbial steroid transformations: current state and prospects. Appl. Microbiol. Biotechnol. 2012; 94, 1423-1447).

### Preparation of steroids

The main intermediate products for the industrial synthesis of steroid drugs are 4-androstene-3,17-dione (hereinafter, AD), 9α-hydroxy-4-androstene-3,17-dione (hereinafter, 9-OH-AD), and 1,4-androstadiene-3,17-dione (hereinafter, ADD). Therefore, cost-effective and efficient production of these intermediates is a pressing need in the pharmaceutical industry.

In the catabolism of microorganisms, 3-ketosteroid-Δ1-dehydrogenase enzyme is responsible for transforming AD into ADD, and 3-ketosteroid 9α-hydroxylase enzyme is responsible for transforming AD into 9-OH-AD.

Enzymes with 3-ketosteroid-Δ1-dehydrogenase activity contain only one type of subunit and are encoded by a single gene generically referred to as *kstD.*

In addition, enzymes described with 3-ketosteroid 9α-hydroxylase activity have two protein subunits and these enzymes are therefore encoded by two different genes, generically referred to as *kshA* (encoding the oxygenase component of the enzyme) and *kshB* (encoding the reductase component of the enzyme), respectively.

One of the main raw materials used in the chemical industry of steroids to obtain the mentioned intermediate products are sapogenins, such as diosgenin. Alternatively, some natural sterols, such as 3β-alcohol steroids containing a double bond at position 5-6 and an aliphatic side chain at C-17, are also used as starting materials in the steroid industry. These steroids include cholesterol, which is known as animal sterol, or phytosterols, which are mixtures of plant-derived sterols, mainly of soybean origin, such as sitosterol, stigmasterol, campesterol and brassicasterol, for example. Since the 1980s, microbial transformation of phytosterol has been a focus of research in the field of steroids *(*Marina V. Donova et al., Microbial steroid transformations: current state and prospects. Appl. Microbiol. Biotechnol. 2012, 94, 1423-1447).

Up until very recently, microbial steroid biotransformation processes were performed using natural microorganisms, either directly without any modification, or using microorganisms mutated by means of conventional methods. Genetic engineering techniques have also been recently applied. Nevertheless, pure AD or ADD has not been successfully produced from cholesterol, phytosterols or other natural sterols up until now.

### Classical mutagenesis and random insertional mutagenesis with transposons

Many publications and patents describe these processes and the microorganisms used. Processes for producing AD and ADD from natural sterols by means of strains of *Mycobacterium* sp. isolated from soil and subsequently mutated by means of UV irradiation have been described, for example (see patent US 3684657 (1972*)*; and William J. Marsheck et al, Microbial Degradation of Sterols, Appl Microbiol., 1972, 23(1), 72-77).

A *Mycobacterium smegmatis* (hereinafter, *M. smegmatis*) insertional mutant blocked in the oxygenase component KshA of 3-ketosteroid 9α-hydroxylase enzyme accumulating mixtures of AD and ADD from β-sitosterol has been obtained by means of random mutagenesis using a transposon (Attila Andor et al., Generation of useful insertionally blocked sterol degradation pathway mutants of fast-growing mycobacteria and cloning, characterization, and expression of the terminal oxygenase of the 3-ketosteroid 9α-hydroxylase in Mycobacterium smegmatis mc2155. Appl. Environ. Microbiol. 2006, 72, 6554-6559*)*

It must be pointed out that mutants which have been isolated using classical mutagenesis or random insertional mutagenesis with transposons are generally unsuitable for industrial processes due to their genetic instability and/or low bioconversion efficiencies. Furthermore, these strains tend to produce mixtures of AD and ADD, and the difficulty in separating these two products is a key bottleneck for microbial transformation of phytosterols in the industry.

### Molecular biology and genetic engineering

New molecular biology and genetic engineering technologies have been applied recently to obtain genetically modified strains capable of accumulating AD, ADD and other intermediate products of steroid metabolism. These techniques are based on knowledge about bacterial genomes metabolizing sterols.

In this sense, several bacterial genomes that metabolize sterols are known, such as the genomes of *M. smegmatis* mc²155 (GenBank CP000480.1), *Mycobacterium tuberculosis* H37Rv (GenBank AL123456.3) or *Rhodococcus jostii* RHA1 (GenBank CP000431.1), for example.

More specifically, some of the genes involved in cholesterol catabolism in some bacteria have been described (Jose L. Garcia et al., Catabolism and biotechnological applications of cholesterol degrading bacterium. Microb. Biotechnol. 2012, 5, 679-699)

A method has also been developed for constructing selective gene deletion mutants in the genus *Rhodococcus* and the *kstD1* gene encoding 3-ketosteroid-Δ1-dehydrogenase (KstD1) enzyme in *Rhodococcus erythropolis* SQ1 was deleted by means of this method, but the mutant strain still has significant residual KstD enzyme activity and did not accumulate intermediate products (Robert van der Geize R et al., Unmarked gene deletion mutagenesis of kstD, encoding 3-ketosteroid Delta1-dehydrogenase, in Rhodococcus erythropolis SQ1 using sacB as counter-selectable marker, FEMS Microbiol Lett. 2001, 18, 205(2), 197-202*).* However, accumulation of 9OH-AD was observed when *kstD1* and *kstD2* genes were inactivated (*Lubbert Dijkhuizen et al., Microbial 9(α)-hydroxylation of steroids, Application of Patent* EP1232278A1).

It has also been demonstrated that it is possible to accumulate ADD from phytosterols in the bacterium *Rhodococcus erythropolis* SQ1 blocked in the two oxygenase (KshA) and reductase (KshB) components of 3-ketosteroid 9α-hydroxylase enzyme by means of genetic engineering through deletion of the two *kshA* and *kshB* genes encoding this enzyme (R. van der Geize, et al., Molecular and functional characterization of kshA and kshB, encoding two components of 3-ketosteroid 9a-hydroxylase, a class IA monooxygenase, in Rhodococcus erythropolis strain SQ1. Molecular Microbiology 2002, 45(4), 1007-1018*) (Robert Van der Geize et al., Identification of 3-ketosteroid 9a-hydroxylase genes and microorganisms blocked in 3-ketosteroid 9α-hydroxylase activity, patent application* WO 03/070925)*.* The problem arising in this case is that the strain has at least three KshA activities and deletion of a single KshA means that the accumulated ADD is metabolized through the action of these additional enzymes (Robert van der Geize, et al., Characterization of a Second Rhodococcus erythropolis SQ1 3-Ketosteroid 9α-Hydroxylase Activity Comprising a Terminal Oxygenase Homologue, KshA2, Active with Oxygenase-Reductase Component KshB. Appl. Environ. Microbiol. 2008, 74, 7197-7203). An additional problem these genetically modified microorganisms present is that ADD can only be accumulated from AD, but not from phytosterols, because production of ADD from natural phytosterols is inhibited for reasons currently unknown.

3-ketosteroid-Δ1-dehydrogenase (KsdD) enzyme activity in the bacterium *Mycobacterium neoaurum* NwlB-01 has been inactivated and augmented also by means of genetic engineering to produce mixtures of AD and ADD from phytosterols *(*Wei Wei et al., Inactivation and Augmentation of the Primary 3-Ketosteroid-Δ1-Dehydrogenase in Mycobacterium neoaurum NwIB-01: Biotransformation of Soybean Phytosterols to 4-Androstene-3,17-Dione or 1,4-Androstadiene-3,17-Dione, Appl. Environ. Microbiol. 2010, 76, 4578-4582).

*M. smegmatis* and *Mycobacterium phlei* mutants that do not grow in β-sitosterol and accumulate mixtures of AD, ADD and other intermediate products in the culture medium in the presence of 3-sitosterol have been found by means of random mutagenesis using transposons (Attila Andor et al., Generation of useful insertionally blocked sterol degradation pathway mutants of fast-growing mycobacteria and cloning, characterization, and expression of the terminal oxygenase of the 3-ketosteroid 9alpha-hydroxylase in Mycobacterium smegmatis mc(2)155. Appl. Environ. Microbiol. 2006, 72, 6554-6559). One of these mutants has been found to have the transposon inserted in the *kshA* gene. The problem these transposition mutants present is that they are very unstable due to the nature of transposons. Furthermore, AD and ADD accumulate in the form of a mixture, making their subsequent separation difficult.

Accumulation of 9α-hydroxy-4-androstene-3,17-dione (9-OH-AD) from AD by means of deletion of 3 *kstD* genes (*kstD1, kstD2 and kstD3*) encoding 3-ketosteroid-Δ1-dehydrogenase (Laura Fernández de las Heras et al., Molecular characterization of three 3-ketosteroid-Δ(1)-dehydrogenase isoenzymes of Rhodococcus ruber strain Chol-4. J. Steroid Biochem. Mol. Biol., 2012, 132, 271-281*)* has recently been demonstrated in an isolated *Rhodococcus ruber* strain Chol-4 (Laura Fernández de las Heras et al., Morphological, Physiological, and Molecular Characterization of a Newly Isolated Steroid-Degrading Actinomycete, Identified as Rhodococcus ruber strain Chol-4. Curr. Microbiol. 2009, 59, 548-553). Nor has it been demonstrated in this case that 9-OH AD can be accumulated from natural sterols with genetically modified bacteria.

*M. smegmatis* mutant strains with one or two genes with *3-ketosteroid-Δ(1)-dehydrogenase (ksdD)* activity that are deleted have been described (Anna Brzostek et al., Identification and targeted disruption of the gene encoding the main 3-ketosteroid dehydrogenase in Mycobacterium smegmatis. Microbiology 2005, 151, 2393-2402). Up to 6 putative genes encoding possible KsdDs have been identified, two of which are selected by homology. Deletion of one of said genes provides a mutant strain capable of accumulating a mixture of AD and 9-OH-AD from cholesterol; nevertheless degradation of AD to ADD is not completely eliminated, confirming the presence of various homologous genes with KsdD activity. Deletion of two genes allows obtaining a mixture of AD and 9-OH-AD without detectable formation of ADD.

Based on the foregoing it is deduced that a recombinant bacterium capable of producing pure AD or ADD from cholesterol, phytosterols or other natural sterols could not be obtained up until now by means of directed mutagenesis.

### DISCLOSURE OF THE INVENTION

The present invention relates to genetically modified *M. smegmatis mc²155* strains with one or more genes involved in cholesterol catabolism being functionally inactivated or completely or partially deleted, as well as to processes for producing substantially pure AD or ADD without them mixing with one another or mixing with 9-OH-AD or 9-OH-ADD, from natural sterols, whether from cholesterol or phytosterols, using said modified strains.

### BRIEF DESCRIPTION OF THE DRAWINGS

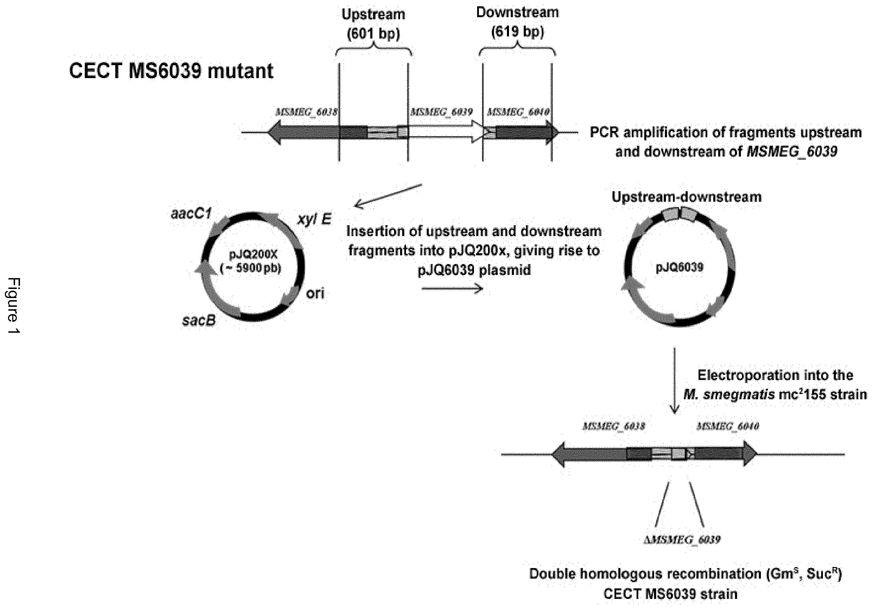

Figure 1 shows the *M. smegmatis* CECT 8331 (MS6039) construct.
   First, two DNA fragments, one in the region upstream of the *MSMEG_6039* gene and another in the region downstream of the same gene, were amplified by means of PCR using the genome of *M. smegmatis* mc²155 as a template. The two generated DNA fragments were digested with restriction enzymes and cloned into the pJQ200x plasmid, generating the pJQ6039 plasmid. The pJQ6039 plasmid was transformed into competent cells of the *M. smegmatis* mc²155 strain by means of electroporation, and a mutant strain selection process was started. The CECT 8331 (MS6039) mutant strain was obtained after two selection steps; simple recombinants (gentamicin-resistant, sucrose-sensitive and *xylE*-positive recombinants) were selected in a first step and they were seeded in a 7H10 agar plate with 10% sucrose to select double recombination events. Sucrose-resistant, gentamicin-sensitive and non *xylE* gene carrier colonies were analyzed by PCR to confirm deletion of *MSMEG_6039,* which meant obtaining the CECT 8331 (MS6039) mutant strain.

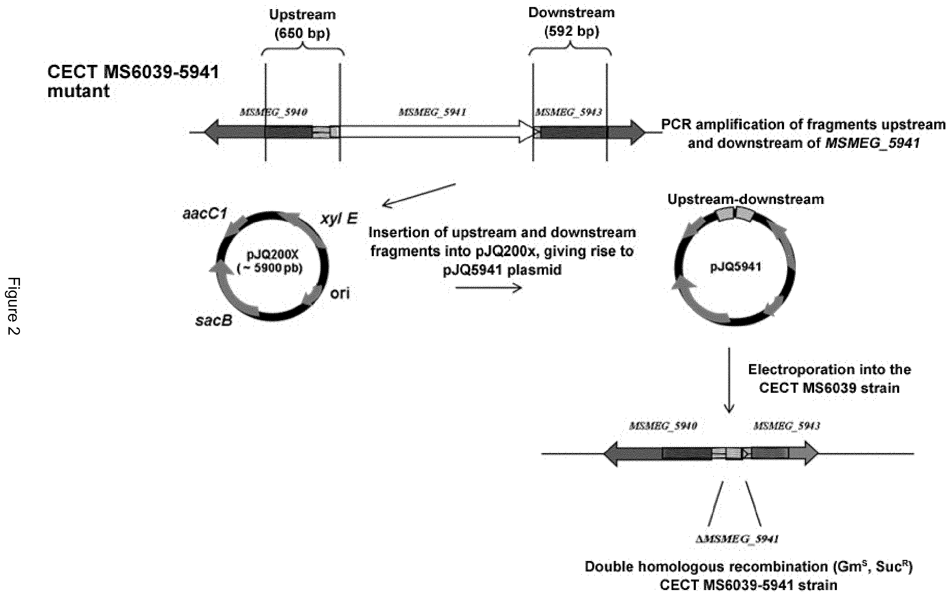

Figure 2 shows the *M. smegmatis* CECT 8332 (MS6039-5941) construct.
   First, two DNA fragments, one in the region upstream of the *MSMEG_5941* gene and another in the region downstream of the same gene, were amplified by means of PCR using the genome of *M. smegmatis* mc²155 as a template. The two generated DNA fragments were digested with restriction enzymes and cloned into the pJQ200x plasmid, generating the pJQ5941 plasmid. The pJQ5941 plasmid was transformed into competent cells of the CECT 8331 (MS6039) strain by means of electroporation, and a new mutant strain selection process was started. The CECT 8332 (MS6039-5941) new mutant strain was obtained after two selection steps; simple recombinants (gentamicin-resistant, sucrose-sensitive and *xylE*-positive recombinants) were selected in a first step, and they were seeded in a 7H10 agar plate with 10% sucrose to select double recombination events. Sucrose-resistant, gentamicin-sensitive and non *xylE* gene carrier colonies were analyzed by PCR to confirm deletion of *MSMEG_5941,* which meant obtaining the CECT 8332 (MS6039-5941) mutant strain.
Figure 3 shows the GC/MS chromatograms showing the production of AD and ADD from cholesterol in CECT MS6039 and CECT MS6039-5941 mutants: A) standards B) ADD in CECT MS6039 and C) AD in CECT MS6039-5941.
Figure 4 shows the GC/MS chromatograms showing the production of AD and ADD from phytosterols in CECT MS6039 and CECT MS6039-5941 mutants: A) standards B) ADD in CECT MS6039 and C) AD in CECT MS6039-5941.
Figure 5 shows growth curves of *M. smegmatis* in cholesterol and in ADD as the only carbon source.
Figure 6 shows the production of AD in a fermenter with the *Mycobacterium smegmatis* CECT 8332 (MS6039-5941) strain using phytosterol (20 g/L): ■ soybean oil; □ methyl-β-cyclodextrin.
Figure 7 shows the production of ADD and AD with the *Mycobacterium smegmatis* CECT 8331 (MS6039) strain using phytosterol (20 g/L): ■ ADD with soybean oil; □ ADD with methyl-β-cyclodextrin; ▲ AD with soybean oil; Δ AD with methyl-β-cyclodextrin.

### DETAILED DISCLOSURE OF THE INVENTION

AD and ADD are used by bacteria as a carbon source when added to a culture medium. Therefore, although mutant AD- and/or ADD-producing bacteria which secrete said substances into the culture medium can be obtained by fermentation, once these compounds are secreted they can be partially metabolized throughout the fermentation time with subsequent losses in the AD and ADD production process yield. This has also been described for *M. smegmatis* (Anna Brzostek et al., Identification and targeted disruption of the gene encoding the main 3-ketosteroid dehydrogenase in Mycobacterium smegmatis. Microbiology 2005, 151, 2393-2402*).*

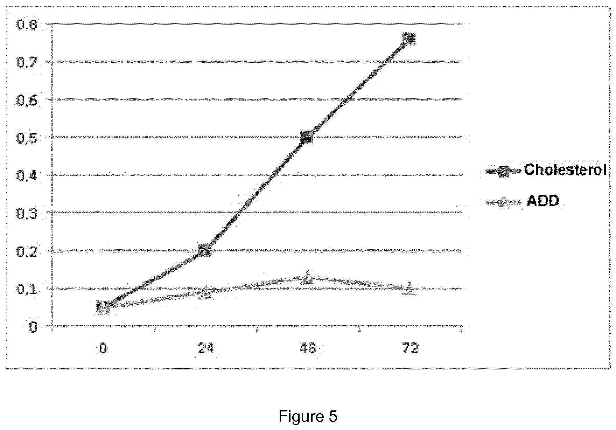

Not withstanding the foregoing, the research conducted by the inventors of the present application has surprisingly determined that wild-type *M. smegmatis* mc²155 bacterium cannot use the substances AD and ADD as a carbon source when said substances are added to a culture medium, despite being intermediate products in bacterial cholesterol catabolism (see Figure 5). This occurs even in the presence of cholesterol which would activate metabolization thereof. This property is not observed in other actinobacteria of the genera *Mycobacterium* or *Rhodococcus* that catabolize cholesterol given that they are capable of growing using AD and ADD as a carbon source.

The bacterium *M. smegmatis* mc²155 is therefore an ideal bacterium for producing AD and ADD since they cannot be catabolized by the metabolism of the bacterium once secreted into the medium.

In addition, according to the hypotheses established concerning the operation of the catabolic pathway for cholesterol described for actinobacteria, it has been proposed that any 3-ketosteroid 9α-hydroxylase activity in the producer cell must be inhibited in order to produce ADD from natural sterols. It has also been proposed that any 3-ketosteroid 9α-hydroxylase activity and any 3-ketosteroid-Δ1-dehydrogenase activity must be inhibited in order to produce AD from natural sterols. Notwithstanding the foregoing, no stable *M. smegmatis* mc²155 strain with inactivated 3-ketosteroid 9α-hydroxylase activity has been described up until now, and much less a stable strain with inactivated 3-ketosteroid 9α-hydroxylase activities and inactivated 3-ketosteroid-Δ1-dehydrogenase activity.

Enzymes with 3-ketosteroid-A1-dehydrogenase activity contain only one type of subunit and are encoded by a single gene generically referred to as *kstD.*

Meanwhile, enzymes described with 3-ketosteroid 9α-hydroxylase activity have two protein subunits, and these enzymes are therefore encoded by two different genes generically referred to as *kshA* (encoding the oxygenase component of the enzyme) and *kshB* (encoding the reductase component of the enzyme), respectively.

It is also known that actinobacteria can have several genes homologous to the *kshA, kshB* or *kstD* genes in the same genome, encoding enzymes with the same activities, so several homologous genes must be eliminated in those cases to completely eliminate the mentioned 3-ketosteroid-Δ1-dehydrogenase and 3-ketosteroid 9α-hydroxylase activities. This has been described for *M. smegmatis (*Anna Brzostek et al., Identification and targeted disruption of the gene encoding the main 3-ketosteroid dehydrogenase in Mycobacterium smegmatis. Microbiology 2005, 151, 2393-2402)*.*

Therefore, it would be expected that inactivation or deletion of a single *kshA or kshB* gene would not lead to complete elimination of 3-ketosteroid 9α-hydroxylase activity in the modified bacterium. Similarly, it would be expected that elimination of a single *kshD* gene would not lead *de facto* to complete elimination of 3-ketosteroid-Δ1-dehydrogenase activity.

To be able to selectively delete a gene, it is essential to first know its DNA sequence. Different computer programs provided with sequence homology analysis algorithms were used to locate said genes using sequences of the *kstH* gene (equivalent to *kstD*) and *orf17* gene (equivalent to *kshB*) of *Comamonas testosteroni* and sequences of the *kstD, kshA1* and *kshB* genes of *Rhodococcus jostii* RHA1, as model sequences. This search showed the presence of *MSMEG_5941* genes and 6 other additional genes encoding possible enzymes with 3-ketosteroid-Δ1-dehydrogenase activity in the bacterium *M. smegmatis.* In addition, *MSMEG_6039* and *MSMEG_2893* genes similar to the *kshB* gene and *MSMEG_5925* and *MSMEG_2870* genes similar to the *kshA* gene were obtained as possible genes encoding a two-component enzyme with 3-ketosteroid 9α-hydroxylase activity in the bacterium *M. smegmatis.*

Once the possible genes responsible for 3-ketosteroid-Δ1-dehydrogenase and 3-ketosteroid 9α-hydroxylase activities were identified, *MSMEG_5941* and *MSMEG_6039* genes were selected due to greater homology, and they were deleted using selective disruption/mutagenesis methods previously described in the literature for actinobacteria (Robert van der Geize R., et al. Unmarked gene deletion mutagenesis of kstD, encoding 3-ketosteroid-Δ1-dehydrogenase, in Rhodococcus erythropolis SQ1 using sacB as counter-selectable marker. FEMS Microbiol. Lett. 2001, 205, 197-202*).*

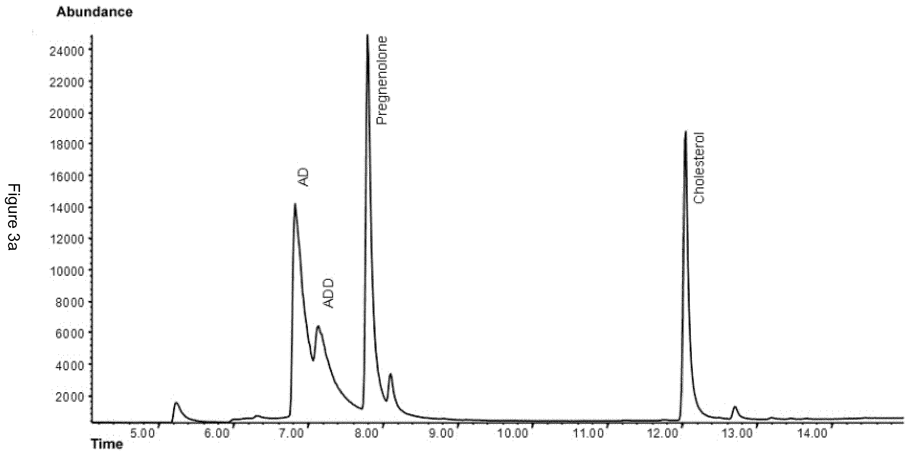

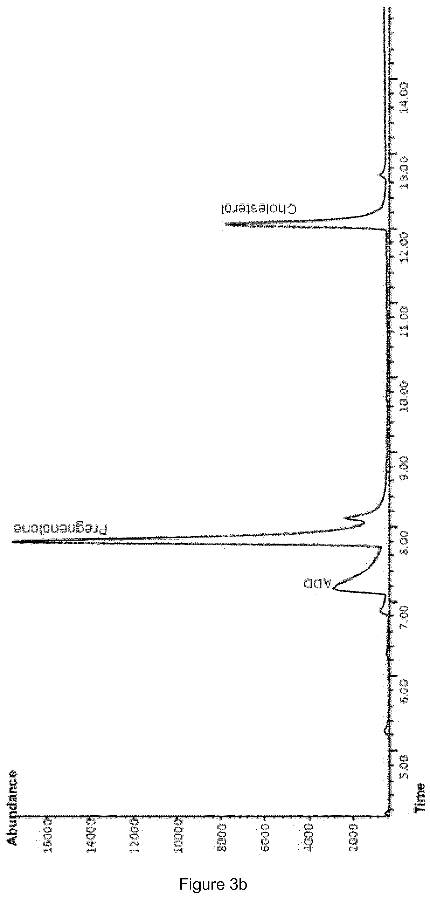

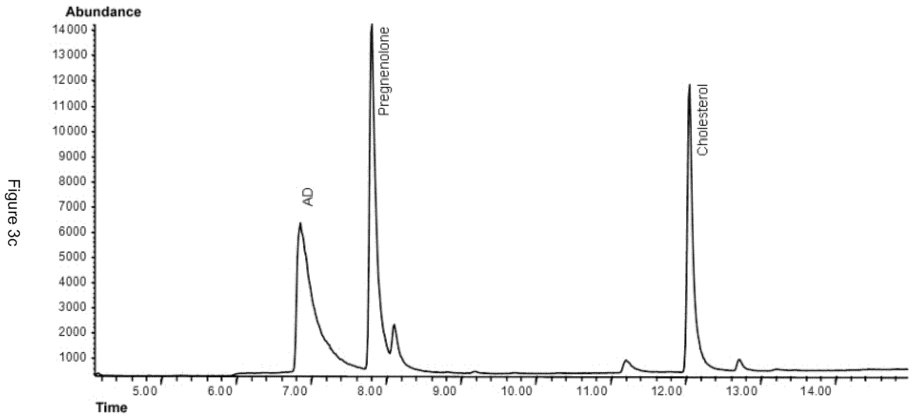

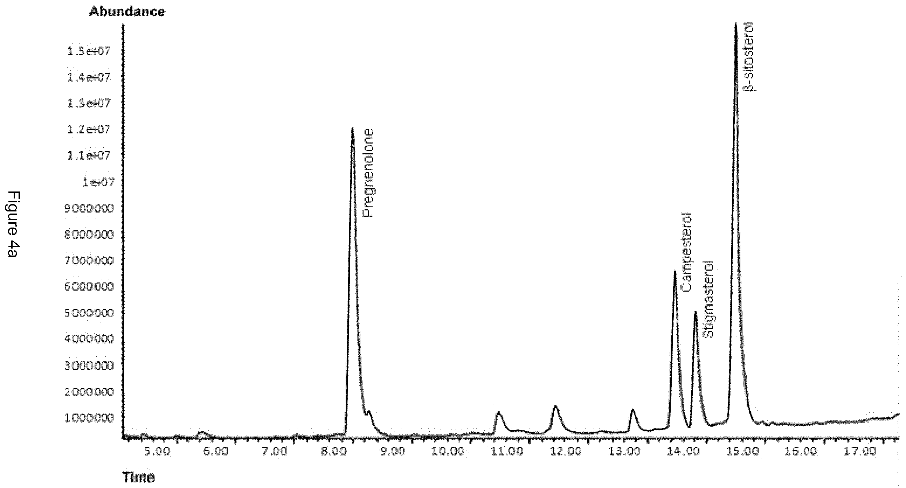

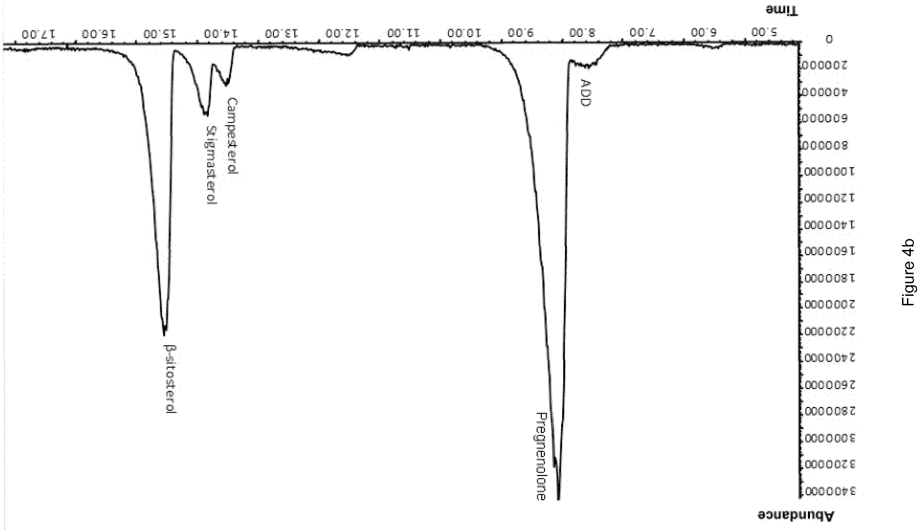

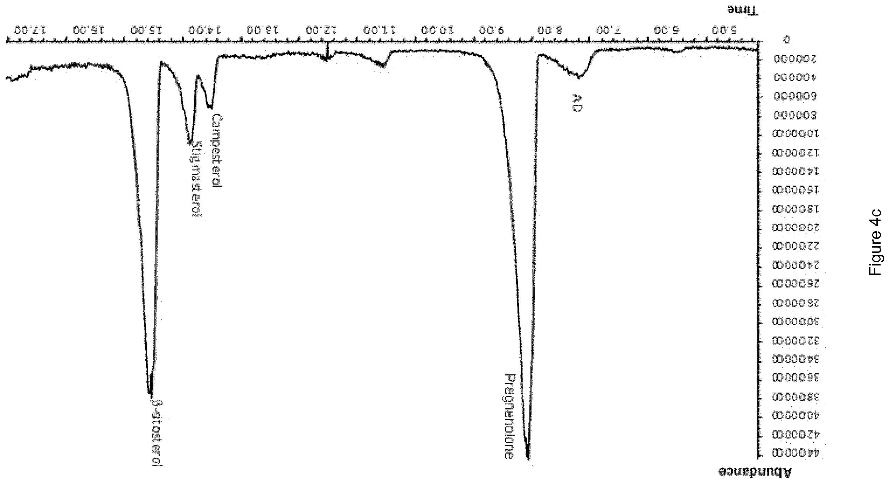

In one embodiment of the present invention, the *MSMEG_6039* gene (*kshB,* encoding the reductase component of 3-ketosteroid 9α-hydroxylase enzyme) was first deleted based on the hypothesis that deletion of this gene would reduce 3-ketosteroid 9α-hydroxylase activity and this would cause the accumulation of the ADD product when the bacterium metabolizes sterols in the presence of an alternative carbon source. The *M. smegmatis* CECT MS6039 mutant having the *MSMEG_6039* gene deleted was therefore constructed. Residual 3-ketosteroid 9α-hydroxylase activity generated by other homologous genes present in the bacterium would be expected. It was surprisingly found that when the *M. smegmatis* CECT MS6039 mutant bacterium was grown in minimal culture medium containing cholesterol or a mixture of phytosterols and an alternative carbon source (such as glycerol, for example), the ADD substance accumulated in the culture medium without a significant presence of AD or 9-OH-AD or 9-OH-ADD (see Figures 3 and 4).

In another embodiment of the present invention, the *MSMEG_5941* (*kstD*) gene in the mutant bacterium *M. smegmatis* CECT MS6039 was deleted. This new mutation was formed considering the hypothesis that deletion of this gene would theoretically reduce 3-ketosteroid-Δ1-dehydrogenase activity, and this along with the fact that the bacterium *M. smegmatis* CECT MS6039 already lacked 3-ketosteroid 9α-hydroxylase activity, would cause the accumulation of the AD product when the bacterium metabolizes sterols in the presence of an alternative carbon source. The *M. smegmatis* CECT MS6039-5941 mutant having the *MSMEG_6039* and *MSMEG_5941* genes deleted was therefore constructed. Residual 3-ketosteroid-Δ-1-dehydrogenase activity generated by other homologous genes present in the bacterium would be expected. It was surprisingly found that when the CECT MS6039-5941 mutant bacterium was grown in minimal culture medium containing cholesterol or a mixture of phytosterols and an alternative carbon source (such as glycerol, for example), the AD substance accumulated in the culture medium without a significant presence of AD or 9-OH-AD or 9-OH-ADD (see Figures 3 and 4).

Therefore, in a first aspect the present invention relates to a first genetically modified strain of the species Mycobacterium smegmatis (M. smegmatis), characterized in that said strain is uncapable of using any of substances AD (4-androstene-3,17-dione) or ADD (1,4-androstadiene-3,17-dione) as a carbon source when said substances are added to a culture medium, and in that in said strain a reductase component of 3-ketosteroid 9 α-hydroxylase enzyme has been functionally inactivated or completely or partially deleted, wherein at least one nucleotide sequence encoding the reductase component of 3-ketosteroid 9α-hydroxylase enzyme corresponds with: a) the sequence having SEQ ID NO 1; or b) a variant to the sequence having SEQ ID NO 1 which is at least 75% homologous to SEQ ID NO 1, based on the identity of all the nucleotides of said sequence, and wherein said strain is capable of producing ADD with a purity of 95% or more with respect to the AD content by fermentation in a minimal culture medium containing cholesterol or a mixture of phytosterols and glycerol as an alternative carbon source to cholesterol.

According to preferred embodiments, the variant to the sequence having SEQ ID NO 1 is preferably at least 80% homologous, even more preferably at least 85% homologous. In other possible embodiments, the variant to the sequence having SEQ ID NO 1 is at least 90%, 95% or 98% homologous.

According to another preferred embodiment, the nucleotide sequence encoding the reductase component of 3-ketosteroid 9α-hydroxylase enzyme corresponds with the sequence having SEQ ID NO 1.

In another preferred embodiment, the nucleotide sequence encoding the reductase component of 3-ketosteroid 9α-hydroxylase enzyme is completely or partially deleted.

The bacterial strain preferably corresponds to the strain deposited in the Spanish Type Culture Collection (located at Parc Científic Universitat de Valencia, Catedrático Agustín Escardino, 9; 46980 Paterna (Valencia), Spain) with accession number CECT 8331 with depositor identification reference number MS6039 on July 4, 2013.

Another aspect of the invention relates to a second genetically modified strain of the species M. smegmatis, characterized in that said strain is uncapable of using any of substances AD or ADD as a carbon source when said substances are added to a culture medium, and in that in said strain at least one nucleotide sequence encoding the reductase component of 3-ketosteroid 9 α-hydroxylase enzyme has been functionally inactivated or completely or partially deleted, and at least one nucleotide sequence encoding 3-ketosteroid-Δ1-dehydrogenase enzyme has been functionally inactivated or completely or partially deleted, wherein the nucleotide sequence encoding the reductase component of 3-ketosteroid 9 α-hydroxylase enzyme corresponds with: a) the sequence having SEQ ID NO 1; or b) a variant to the sequence having SEQ ID NO 1 which is at least 75% homologous to SEQ ID NO 1, based on the identity of all the nucleotides of said sequence, and wherein the nucleotide sequence encoding 3-ketosteroid-Δ1-dehydrogenase enzyme corresponds with: a) the sequence having SEQ ID NO 2; or b) a variant to the sequence having SEQ ID NO 2 which is at least 75% homologous to SEQ ID NO 2, based on the identity of all the nucleotides of said sequence, and wherein said genetically modified strainis capable of producing AD with a purity of 95% or more with respect to the ADD content by fermentation in a minimal culture medium containing cholesterol or a mixture of phytosterols and glycerol as an alternative carbon source to cholesterol.
Said sequence even more preferably corresponds with the sequence having SEQ ID NO 2.

According to preferred embodiments, the variant to the sequence having SEQ ID NO 2 is preferably at least 80% homologous, even more preferably at least 85% homologous. In other possible embodiments, the variant to the sequence having SEQ ID NO 2 is at least 90%, 95% or 98% homologous.

According to preferred embodiments, the variant to the sequence having SEQ ID NO 1 is preferably at least 80% homologous, even more preferably at least 85% homologous. In other possible embodiments, the variant to the sequence having SEQ ID NO 1 is at least 90%, 95% or 98% homologous. Likewise, the variant to the sequence having SEQ ID NO 2 is preferably at least 80% homologous, even more preferably at least 85% homologous. In other possible embodiments, the variant to the sequence having SEQ ID NO 2 is at least 90%, 95% or 98% homologous.

The bacterial strain more preferably corresponds to the strain deposited in the Spanish Type Culture Collection (located at Parc Científic Universitat de Valencia, Catedrático Agustín Escardino, 9, 46980 Paterna (Valencia), Spain) with accession number CECT 8332 with depositor identification reference MS6039-5941 on July 4, 2013.

An additional aspect of the invention relates to the use of the first bacterial strain defined above for producing substantially pure ADD by means of fermenting natural sterols. The natural sterols are preferably selected from cholesterol or phytosterols. The natural sterols can only be cholesterol or a phytosterol, or a mixture of phytosterols.

Another additional aspect of the invention relates to the use of the second bacterial strain defined above, i.e., the double mutant, for producing substantially pure AD by means of fermenting natural sterols. The natural sterols are preferably selected from cholesterol or phytosterols. The natural sterols can only be cholesterol or a phytosterol, or a mixture of phytosterols.

Another aspect of the invention relates to a method for obtaining ADD with a purity of 95% or more with respect to the AD content in a minimal culture medium containing cholesterol or a mixture of phytosterols and an alternative carbon source to cholesterol such as glycerol, characterized in that it comprises the steps of:
a. preparing and sterilizing a mixture of a natural sterol or a mixture of natural sterols in a polyalcohol or a vegetable oil, with the addition of mineral salts;
b. growing a culture of the strain comprising at least one nucleotide sequence encoding the reductase component of 3-ketosteroid 9α-hydroxylase enzyme that has been functionally inactivated or completely or partially deleted, and in that it is capable of producing substantially pure 1,4-androstadiene-3,17-dione (ADD) by fermentation, as defined above;
c. separating the cell pellet to obtain a solution containing ADD.

According to a particular embodiment, a surfactant is added in step a).

This method can be carried out both at the laboratory level and the industrial level, preferably at the industrial level.

Other preferred embodiments are shown in Examples 8 and 9, particularly in reference to preparing culture media.

An additional aspect of the invention relates to a method for obtaining AD with a purity of 95% or more with respect to the ADD content in a minimal culture medium containing cholesterol or a mixture of phytosterols and an alternative carbon source to cholesterol such as glycerol, characterized in that it comprises the steps of:
a. preparing and sterilizing a mixture of a natural sterol or a mixture of natural sterols in a polyalcohol or a vegetable oil, with the addition of mineral salts;
b. growing a culture of the strain comprising at least one nucleotide sequence encoding the reductase component of 3-ketosteroid 9α-hydroxylase enzyme that has been functionally inactivated or completely or partially deleted, and at least one nucleotide sequence encoding 3-ketosteroid-Δ1-dehydrogenase enzyme that has been functionally inactivated or completely or partially deleted; and in that it is capable of producing substantially pure 4-androstene-3,17-dione (AD) by fermentation, as defined above, in the mixture obtained in a);
c. separating the cell pellet to obtain a solution containing AD.

According to a particular embodiment, a surfactant is added in step a).

This method can be carried out both at the laboratory level and the industrial level, preferably at the industrial level.

Other preferred embodiments are shown in Examples 6 and 7, particularly in reference to preparing culture media and the composition thereof.

According to another aspect, the invention relates to a method for obtaining ADD with a purity of 95% or more with respect to the AD content in a minimal culture medium containing cholesterol or a mixture of phytosterols and an alternative carbon source to cholesterol such as glycerol, characterized in that it comprises the steps of:
a. preparing a mixture of a natural sterol or a mixture of natural sterols in a polyalcohol or a vegetable oil;
b. preparing a culture of the strain comprising at least one nucleotide sequence encoding the reductase component of 3-ketosteroid 9α-hydroxylase enzyme that has been functionally inactivated or completely or partially deleted, and in that it is capable of producing substantially pure 1,4-androstadiene-3,17-dione (ADD) by fermentation, as defined above;
c. incubating the mixture from a) with the cell culture from b);
d. separating the cell pellet to obtain a solution containing ADD.

According to a preferred embodiment, the mixture from a) is sterilized before being used in step c). This step is known to the person skilled in the art.

The polyalcohol of step a) is preferably selected from the group consisting of methyl-beta-cyclodextrin, polyethylene glycol, ethylene glycol, sorbitan monostearate, sorbitan monopalmitate, or mixtures thereof, or mixtures with other polyalcohols.

The vegetable oil of step a) is preferably selected from soybean oil, sunflower oil, rapeseed oil, olive oil, palm oil, jatropha oil and coconut oil, or mixtures thereof, or mixtures with other vegetable oils.

Other preferred embodiments are shown in Examples 8 and 9, particularly in reference to preparing culture media and the composition thereof.

Mineral salts such as ZnSO₄, FeSO₄, MnSO₄, CuSO₄, CoSO₄, H₃PO₃, and KI are preferably added to the mixture of a).

A surfactant can preferably be incorporated in step a). According to a particular embodiment, said surfactant is selected from polysorbate 80 (Tween 80™) or polysorbate 20 (Tween 20™).

Step b) of preparing a cell or bacterial culture can be carried out in media known to the person skilled in the art, or even in commercial media, such as rich Middlebrook 7H9 medium (Difco), preferably supplemented with albumin-dextrose-catalase (Becton Dickinson). Alternatively, a culture medium incorporating, for example, one or more molasses, a vegetable oil such as soybean oil, for example, NaNO₃ and NH₄H₂PO₄, can be prepared. The culture temperature is preferably between 30 and 44°C, more preferably between 34 and 40°C, even more preferably between 36 and 38°C.

The incubation medium of step c) can comprise components such as soybean oil, methyl-beta-cyclodextrin, NH₄NO₃, KH₂PO₄, K₂HPO₄, KCl or CaCl₂. A mixture of micronutrients incorporating at least one of ZnSO₄, FeSO₄, MnSO₄, CuSO₄, CoSO₄, H₃PO₃ and KI is also preferably added. The incubation temperature is preferably between 30 and 44°C, more preferably between 34 and 40°C, even more preferably between 36 and 38°C.

According to another particular embodiment, a cell pellet can be obtained before step c) of incubation according to techniques known to the person skilled in the art.

According to a preferred embodiment, the method for obtaining substantially pure ADD comprises the steps of:
a) preparing a mixture of a natural sterol or a mixture of natural sterols in a polyalcohol in the presence of a surfactant;
b) preparing a culture of the first bacterial strain defined above and obtaining a cell pellet therefrom;
c) incubating the mixture from a) with the cell pellet from b);
d) separating the cell pellet to obtain a solution containing substantially pure ADD.

The invention also relates to substantially pure ADD obtainable by means of the preceding method, as well as to the use of said ADD for preparing steroids. Another aspect of the invention relates to a steroid obtainable from said ADD. An additional aspect of the invention relates to a steroid obtained from said ADD.

Methodologies and processes for producing steroids using ADD as a starting product are known in the state of the art. Such processes and methodologies are described, for example, in publications such as The Organic Chemistry of Drug synthesis, vol 1, Lednicer D. Mitscher L, Wiley 1977*;* Encyclopedia of Pharmaceutical substances, Kleeman and Engels, Thieme Verlag, 2001*;* and Organic reactions in Steroid Chemistry vol II, Fried J, Edwards J, Van Nostrand Reinhold, 1972*.* Such steroids obtainable according to the present invention are preferably those which are part of the estrogen, androgen, progestogen and glucocorticoid families, for example, estrone, estradiol, testosterone or fluoxymesterone, among others.

Another aspect of the invention relates to a method for obtaining AD with a purity of 95% or more with respect to the ADD content in a minimal culture medium containing cholesterol or a mixture of phytosterols and an alternative carbon source to cholesterol such as glycerol, characterized in that it comprises the steps of:
a. preparing a mixture of a natural sterol or a mixture of natural sterols in a polyalcohol or a vegetable oil;
b. preparing a culture of the strain comprising at least one nucleotide sequence encoding the reductase component of 3-ketosteroid 9α-hydroxylase enzyme that has been functionally inactivated or completely or partially deleted, and at least one nucleotide sequence encoding 3-ketosteroid-Δ1-dehydrogenase enzyme that has been functionally inactivated or completely or partially deleted; and in that it is capable of producing substantially pure 4-androstene-3,17-dione (AD) by fermentation, as defined above;
c. incubating the mixture from a) with the cell culture from b);
d. separating the cell pellet to obtain a solution containing AD.

According to a preferred embodiment, the mixture of a) is sterilized before being used in step c). This step is known to the person skilled in the art.

The polyalcohol of step a) is preferably selected from the group consisting of methyl-beta-cyclodextrin, polyethylene glycol, ethylene glycol, sorbitan monostearate, sorbitan monopalmitate, or mixtures thereof, or mixtures with other polyalcohols.

The vegetable oil is preferably selected from soybean oil, sunflower oil, rapeseed oil, olive oil, palm oil, jatropha oil and coconut oil, or mixtures thereof, or mixtures with other vegetable oils.

Other preferred embodiments are shown in Examples 6 and 7, particularly in reference to preparing culture media and the composition thereof.

Mineral salts such as ZnSO₄, FeSO₄, MnSO₄, CuSO₄, CoSO₄, H₃PO₃, and KI are preferably added to the mixture of a).

Furthermore, a surfactant can optionally be incorporated in step a). According to a particular embodiment, said surfactant is selected from polysorbate 80 (Tween 80™) or polysorbate 20 (Tween 20™).

Step b) of preparing a cell or bacterial culture can be carried out in media known to the person skilled in the art, or even in commercial media, such as rich Middlebrook 7H9 medium (Difco), preferably supplemented with albumin-dextrose-catalase (Becton Dickinson). Alternatively, a culture medium incorporating, for example, one or more molasses, a vegetable oil such as soybean oil, for example, NaNO₃ and NH₄H₂PO₄, can be prepared. The culture temperature is preferably between 30 and 44°C, more preferably between 34 and 40°C, even more preferably between 36 and 38°C.

The incubation medium of step c) can comprise components such as soybean oil, methyl-beta-cyclodextrin, NH₄NO₃, KH₂PO₄, K₂HPO₄, KCl or CaCl₂. A mixture of micronutrients incorporating at least one of ZnSO₄, FeSO₄, MnSO₄, CuSO₄, CoSO₄, H₃PO₃ and KI is also preferably added. The incubation temperature is preferably between 30 and 44°C, more preferably between 34 and 40°C, even more preferably between 36 and 38°C.

According to another particular embodiment, a cell pellet can be obtained before step c) of incubation according to techniques known to the person skilled in the art. According to a preferred embodiment, the method for obtaining substantially pure AD comprises the steps of:
a) preparing a mixture of a natural sterol or a mixture of natural sterols in a polyalcohol in the presence of a surfactant;
b) preparing a culture of the previously defined second bacterial strain and obtaining a cell pellet therefrom;
c) incubating the mixture from a) with the cell pellet from b);
d) separating the cell pellet to obtain a solution containing substantially pure AD.

The invention also relates to substantially pure AD obtainable by means of the preceding method, as well as to the use of said AD for preparing steroids. Another aspect of the invention relates to a steroid obtainable from said AD. An additional aspect of the invention relates to a steroid obtained from said AD. Methodologies and processes for producing steroids using AD as the starting product are known in the state of the art. Such processes and methodologies are described, for example, in publications such as The Organic Chemistry of Drug synthesis, vol 1, Lednicer D. Mitscher L, Wiley 1977*;* Encyclopedia of Pharmaceutical substances, Kleeman and Engels, Thieme Verlag, 2001*;* and Organic reactions in Steroid Chemistry vol II, Fried J, Edwards J, Van Nostrand Reinhold, 1972*.* Such steroids obtainable according to the present invention are preferably those which are part of the estrogen, androgen, progestogen and glucocorticoid families, for example, estrone, estradiol, testosterone or fluoxymesterone, among others.

As it is used herein, the term "deletion of a gene" refers to the complete or partial elimination of a gene by means of completely or partially eliminating the DNA sequence characterizing that gene in the genome of a bacterium.

As it is used herein, the term "nucleotide sequence that has been functionally inactivated" relates to a nucleotide sequence that is not capable of performing its functionality, i.e., it is not capable of providing a functional enzyme.

As it is used herein, the term "homologous sequence" refers to a sequence having the indicated % of homology based on the identity of all the nucleotides of said sequence.

In the context of the present invention, the term "substantially pure" refers to a purity of 95% or more, preferably 97% or more, even more preferably 99% or more, more preferably 100% of the product obtained with respect to the AD content in the case of obtaining ADD and with respect to the ADD content in the case of obtaining AD.

As it is used herein, the term "catabolism" refers to all enzymatic processes leading to the transformation of a complex substance into other simpler substances which are incorporated into the bacterial biomass or eliminated into the environment.

The following examples serve to illustrate but do not, in any case, limit the present invention.

### EXAMPLES

### Example 1: Identification of the kshA, kshB and kstD genes in M. smegmatis mc²155

To identify the *kshA, kshB* and *kstD* genes in M. *smegmatis* mc²155, the genes of C. *testosteroni* TA441 or *R. jostii* RHA1 were compared *in silico* to the genome of M. *smegmatis* mc²155. The BLAST program (http://www.ncbi.nlm.nih.gov/pubmed) was used to that end.

Gene sequences encoding 3-ketosteroid-Δ1-dehydrogenase and 3-ketosteroid 9-α-hydroxylase enzymes of *C. testosteroni* TA441 are deposited in the DDBJ, EMBL and GenBank sequence databases with accession number AB076368, whereas the corresponding genome sequences of *R. jostii* RHA1 are deposited in the GenBank sequence database with accession number CP000431.1.

Once the sequences of *M. smegmatis* were identified, the percentage of similarity between the corresponding protein sequences was determined by means of the ClustalW2 program (http://www.ebi.ac.uk/Tools/msa/clustalw2).

Sequences identified in *M. smegmatis* and percentages of similarity are shown in Tables 1 and 2.

Tables 1 and 2. *In silico* comparison of the genes of *C. testosteroni* TA441 or *R. jostii* RHA1 with respect to the genome of *M. smegmatis* mc²155. The sequences were identified by means of the BLAST program. The percentage of identity (%) found in each case between protein sequences with greater similarity is indicated using the ClustalW2 program. The sequences of the probes used with respect to *C. testosteroni* TA441 are deposited in the DDBJ, EMBL and GenBank sequence databases with accession number AB076368. The genome sequence of *R. jostii* RHA1 is deposited in the GenBank sequence database with accession number CP000431.1.

**Table 1**

| Function | Genes in *Comamonas testosteroni* TA441 | Similar genes in *Mycobacterium smegmatis* mc²155 | % identity (clustalW) |
|---|---|---|---|
| 3-ketosteroid-Δ1-dehydrogenase (KstD) | *tesH* | *MSMEG_5941* | 36 |
| | | *MSMEG_4864* | 33 |
| 3-ketosteroid 9-α-hydroxylase, reductase component (KshB) | *orf17* | *MSMEG_6039* | 44 |
| | | *MSMEG_2893* | 43 |

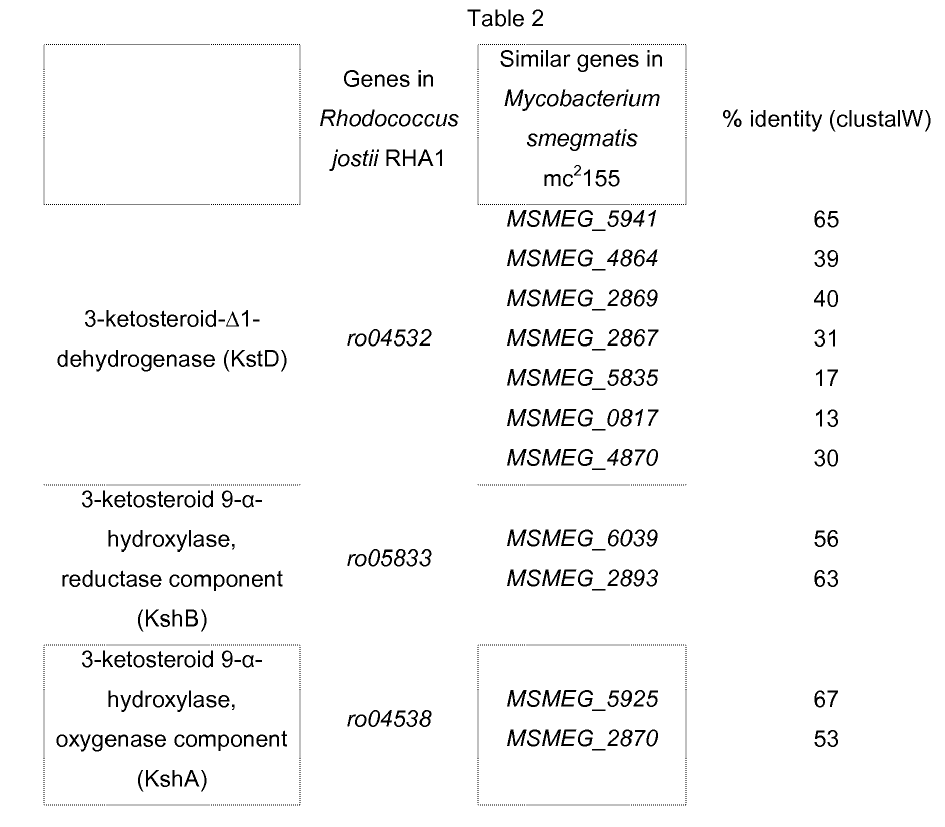

### Example 2: M. smegmatis CECT 8331 (MS6039) mutant strain construct

The CECT MS6039 strain was constructed by means of homologous recombination using the pJQ200x plasmid, a derivative of the pJQ200 suicide vector that does not replicate in Mycobacterium (Jackson, M., Camacho, L.R., Gicquel, B., and Guilhot, C. (2001) Gene replacement and transposon delivery using the negative selection marker sacB*.* Mycobacterium tuberculosis Protocols. Parish, T., and Stoker, N.G. (eds). Totowa, NJ, USA: Humana Press Inc, pp. 59-75.). The strategy consists of PCR amplification of two fragments of -600 base pairs, one fragment containing a region upstream of the *MSMEG_6039* gene (of -500 base pairs) and -100 base pairs of the 5' end of the gene (using the MSMEG_6039 Up F oligonucleotide: CTAGCTCGAGCCAGTTGTGCACACCGATG (SEQ ID NO 3), and the MSMEG_6039 Up R oligonucleotide: CTAGACTAGTGCAGCGTCAGGAACTGGC (SEQ ID NO 4)), and the second fragment containing a region downstream of the gene (of -500 base pairs) and -100 base pairs of the 3' end of the gene (using the MSMEG_6039 Down F oligonucleotide: CTAGACTAGTGGTGCACATGGAGATCAACG (SEQ ID NO 5), and the MSMEG_6039 Down R oligonucleotide: CTAGTCTAGAGTACCAGTCGATCGGTGTC (SEQ ID NO 6)). The two generated fragments were digested with the corresponding enzymes and cloned into the pJQ200x plasmid, generating the pJQ6039 plasmid. pJQ6039 was subjected to electroporation into competent cells of *M. smegmatis* mc²155 to obtain the MS6039 strain. Simple recombination events were selected in 7H10 agar plates (Difco) containing 5 µg ml⁻¹ of gentamicin to which this plasmid confers resistance, and the presence of the *xylE* gene encoded in pJQ200x was confirmed by means of adding catechol on individual colonies. The appearance of a yellow color indicates the presence of the *xylE* gene. The colonies were also counter-selected in 10% sucrose, making cells in which pJQ200x has recombined non-viable, due to the presence of the *sacB* gene conferring sensitivity to sucrose in the plasmid. A single gentamicin-resistant, catechol-positive and sucrose-sensitive colony was cultured in 10 ml of 7H9 medium with 5 µg ml⁻¹ of gentamicin to an optical density of 0.8-0.9. 20 µl of a 1:100 dilution were seeded in a 7H10 agar plate with 10% sucrose for selecting double recombination events. Possible double recombinants (sucrose-resistant recombinants) were selected to check their sensitivity to gentamicin and the absence of the *xylE* gene. Sucrose-resistant, gentamicin-sensitive and non *xylE* gene carrier colonies were analyzed by PCR to confirm the elimination of *MSMEG_6039,* which meant obtaining the MS6039 mutant strain.

### Example 3: Production of ADD with the M. smegmatis CECT 8331 (MS6039) mutant strain from sterols

The production media for obtaining ADD in this example are formed by a mixture of 18 mM glycerol and 1.8 mM cholesterol or a mixture of 18 mM glycerol - mixture of phytosterols. The mixture of phytosterols contains (percentage w/w): brassicasterol (2.16%), stigmasterol (14.69%), campesterol (25.19%) and β-sitosterol (53.07%). The amount of the mixture of phytosterols added to the production medium is that in which β-sitosterol is at a concentration of 1 mM. The cholesterol or phytosterols were dissolved in 10% or 5% Tyloxapol, respectively, before being added to the minimal medium with glycerol. Due to the low solubility of cholesterol and phytosterols, the stock solutions of these compounds were heated at 80°C under stirring in an ultrasound bath for 1 hour and then sterilized in an autoclave.

For ADD production assays, the CECT MS6039 strain was first cultured in 10 ml of rich Middlebrook 7H9 medium (Difco) supplemented with 10% albumin-dextrose-catalase (Becton Dickinson) for 24 hours at a temperature of about 37°C. The cultures were centrifuged and washed. The cell pellets were used for inoculating 20 ml of the production media at an initial OD₆₀₀ of 0.05 and incubated for 120 hours. Aliquots of the cultures (2 ml) were extracted at different incubation times for liquid chromatography-mass spectrometry (GC/MS) analysis as explained below.

For GC/MS analysis, aliquots of the cultures (2 ml) were extracted at different incubation times with the same volume of chloroform. This chloroform fraction was then concentrated by evaporation. 50 µl of a 20 mg/ml solution containing pregnenolone in chloroform was added to the aliquots as the internal standard before extraction with chloroform. The concentrated chloroform fractions were analyzed by GC/MS as described below:
The dry samples were processed in order to obtain the corresponding trimethylsilyl-derivatives. To that end, 50 µL of pyridine (Merck) and 50 µL of N,O-Bis(trimethylsilyl)trifluoroacetamide (BSTFA, Fluka) were added to each sample, incubating at 60°C for 45 minutes in a thermoblock. The trimethylsilyl-derivatives were identified and quantified by means of gas chromatography-mass spectrometry (GC-MS). The analyses were performed in Agilent 7980A-5975C equipment using an HP-5MS column (Agilent, 30 m x 0.25 mm, film thickness of 0.2 µm) and helium (22 psi) as the carrier gas. The split/splitless injector was programmed at a temperature of 300°C, injecting 1 µL of sample with a 20:1 split ratio for analysis. A temperature program that starts isothermally at 240°C (3 minutes) was used for separating the trimethylsilyl-derivatives, subsequently applying a temperature ramp of 5°C min⁻¹ until a final temperature of 300°C. The compounds were detected, simultaneously recording the chromatograms in full scan mode (m/z range of 50-550) and SIM (selective ion monitoring) mode. In the SIM mode, ions characteristic of each of the compounds of interest were exclusively detected: m/z 458 for cholesterol, 384 for cholestenone, 286 for AD and 284 for ADD. The peak of each analyte in the chromatogram was identified by comparing its retention time and mass spectrum with those of the standards analyzed under the same conditions. Quantification was performed using Agilent GC-ChemStation Rev.B.04.02 (96*)* software, considering the area of the peaks detected in SIM mode, applying the response factors previously calculated for each compound with respect to the internal standard (cholestenone).

When cholesterol was used as a steroid source, an accumulation of 1.2 mM of ADD in the culture medium was obtained in 72 hours, which is equivalent to transformation of 50% of the supplied cholesterol. The GC/MS chromatogram can be seen in Figure 3b.

When phytosterols were used as a steroid source, an accumulation of 1.3 mM of ADD in the culture medium was obtained in 96 hours, which is equivalent to transformation of 50% of the supplied phytosterols. The GC/MS chromatogram can be seen in Figure 4b.

### Example 4: M. smegmatis CECT 8332 (MS6039-5941) mutant strain construct

The CECT MS6039-5941 strain was constructed by means of homologous recombination using the pJQ200x plasmid on the already mutated MS6039 strain. Two fragments of -600 base pairs were amplified by PCR, one fragment containing a region upstream of the *MSMEG_5941* gene (of ∼ 500 base pairs) and -100 base pairs of the 5' end of the gene (using the MSMEG_5941 Up F oligonucleotide: CTAGACTAGTGATGTTGCGAATGTCG ATGTC (SEQ ID NO 7), and the MSMEG_5941 Up R oligonucleotide: CTAGTCTAGACCACCACAACGTCGTACTCC (SEQ ID NO 8)), and the second fragment containing a region downstream of the gene (of ∼500 base pairs) and -100 base pairs of the 3' end of the gene (using the MSMEG_5941 Down F oligonucleotide: CTAGTCTAGACCATGACATT CGGTTACCTGG (SEQ ID NO 9) and the MSMEG_5941 Down R oligonucleotide: CTAGGAGCTCGCAGGGA GATCTCGAAATCG (SEQ ID NO 10)). The two generated fragments were digested with the corresponding enzymes and cloned into the pJQ200x plasmid, generating the pJQ5941 plasmid. pJQ5941 was subjected to electroporation into competent cells of the MS6039 strain to obtain the MS6039-5941 strain. Simple recombination events were selected in 7H10 agar plates containing 5 µg ml⁻¹ of gentamicin, and the presence of the *xylE* gene encoded in pJQ200x was confirmed by means of adding catechol on individual colonies. The colonies were also counter-selected in 10% sucrose. A single gentamicin-resistant, catechol-positive and sucrose-sensitive colony was cultured in 10 ml of 7H9 medium with 5 µg ml⁻¹ of gentamicin to an optical density of 0.8-0.9. 20 µl of a 1:100 dilution were seeded in a 7H10 agar plate with 10% sucrose for selecting double recombination events. Possible double recombinants (sucrose-resistant recombinants) were selected to check their sensitivity to gentamicin and the absence of the *xylE* gene. Sucrose-resistant, gentamicin-sensitive and non *xylE* gene carrier colonies were analyzed by PCR to confirm the elimination of *MSMEG_5941,* which meant obtaining the MS6039-5941 mutant strain.

### Example 5: Production of AD with the M. smegmatis CECT 8332 (MS6039-5941) mutant strain from sterols

The production media for obtaining AD are the same as those used for obtaining ADD (EXAMPLE 3).

For AD production assays, the CECT MS6039-5941 strain was first cultured in 10 ml of rich Middlebrook 7H9 medium supplemented with 10% albumin-dextrose-catalase for 24 hours. The cultures were centrifuged and washed. The cell pellets were used for inoculating 20 ml of the production medium at an initial OD₆₀₀ of 0.05 and incubated for 120 hours. Aliquots of the cultures (2 ml) were extracted at different incubation times for gas chromatography-mass spectrometry (GC/MS) analysis as explained below.

For GC/MS analysis, aliquots of the cultures (2 ml) were extracted at different incubation times with the same volume of chloroform. This chloroform fraction was then concentrated by evaporation. 50 µl of a 20 mg/ml solution containing pregnenolone in chloroform was added to the aliquots as the internal standard before extraction with chloroform. The concentrated chloroform fractions were analyzed by GC/MS as described in Example 3.

When cholesterol was used as a steroid source, an accumulation of 1.4 mM of AD in the culture medium was obtained in 72 hours, which is equivalent to transformation of 80% of the supplied cholesterol. The GC/MS chromatogram can be seen in Figure 3c.

When phytosterols were used as a steroid source, an accumulation of 0.9 mM of AD in the culture medium was obtained in 96 hours, which is equivalent to transformation of 44% of the supplied phytosterols. The GC/MS chromatogram can be seen in Figure 4c.

### Example 6. Production of AD in a fermenter with the Mycobacterium smegmatis CECT 8332 (MS6039-5941) strain using phytosterol and vegetable oil

Fermentation was performed in 2 phases using a vegetative inoculum and fermenter. The vegetative inoculum was seeded with 1 ml of microorganism (about 10⁸ cells) and incubated in a 5 L fermenter with 3 L of culture medium with the following composition: 54 g/L of molasses, 5.4 g/L of NaNo₃, 0.6 g/L of NH₄H₂PO₄, and 20 g/L of soybean oil, all adjusted to pH 7.0. The vegetative inoculum was incubated for 48-72 hours at 37°C with aeration of 0.5 volume/volume/minute (vvm), relative pressure of 0.5 barg and stirring at 150 rpm. Cell pellet volumes of 5-20% analyzed by centrifugation for 10 minutes at 2,000 x g were thereby obtained.

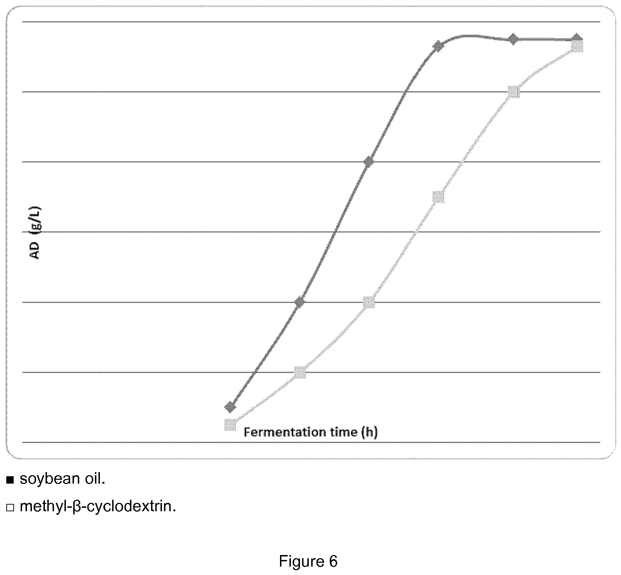

Fermentation (biotransformation) was performed in a 42 L fermenter with 30 L of culture medium with the following composition (g/L): 2 g/L of NH₄NO₃, 1 g/L of KH₂PO₄, 2 g/L of K₂HPO₄, 0.2 g/L of KCI, 0.3 g/L of CaCl₂, 150 g/L of soybean oil, and 20-40 g/L of phytosterol; 1 mL of a mixture of micronutrients was additionally added for each L of fermentation medium. The composition of the mixture of micronutrients is as follows: 11 g/L of ZnSO₄, 1 g/L of FeSO₄, 6 g/L of MnSO₄, 0.04 g/L of CuSO₄, 0.3 g/L of CoSO₄, 0.03 g/L of H₃PO₃, and 0.001 g/L of KI, all adjusted to pH 7.0. The fermenter was seeded with 3 L of vegetative inoculum and incubated for 120-192 hours at 37°C, with aeration of 0.5 volume/volume/minute (vvm), relative pressure of 0.5 barg and stirring at 150 rpm. The obtained results are shown in Figure 6.

### Example 7. Production of AD in a fermenter with the Mycobacterium smegmatis CECT 8332 (MS6039-5941) strain using phytosterol and methyl-β-cyclodextrin

Fermentation was performed in 2 phases using a vegetative inoculum and fermenter. The vegetative inoculum was seeded with 1 ml of microorganism (about 10⁸ cells) and incubated in a 5 L fermenter with 3 L of culture medium with the following composition: 54 g/L of molasses, 5.4 g/L of NaNo₃, 0.6 g/L of NH₄H₂PO₄, and 20 g/L of soybean oil, all adjusted to pH 7.0. The vegetative inoculum was incubated for 48-72 hours at 37°C, with aeration of 0.5 volume/volume/minute (vvm), relative pressure of 0.5 barg and stirring at 150 rpm. Cell pellet volumes of 5-20% analyzed by centrifugation for 10 minutes at 2,000 x g were thereby obtained.

Fermentation (biotransformation) was performed in a 42 L fermenter with 30 L of culture medium with the following composition (g/L): 2 g/L of NH₄NO₃, 1 g/L of KH₂PO₄, 2 g/L of K₂HPO₄, 0.2 g/L of KCI, 0.3 g/L of CaCl₂, 40 g/L of methyl-β-cyclodextrin (Cavasol® W7M, Wacker Chemie), and 20 g/L of phytosterol; 1 mL of a mixture of micronutrients was additionally added for each L of fermentation medium. The composition of the mixture of micronutrients is as follows: 11 g/L of ZnSO₄, 1 g/L of FeSO₄, 6 g/L of MnSO₄, 0.04 g/L of CuSO₄, 0.3 g/L of CoSO₄, 0.03 g/L of H₃PO₃, and 0.001 g/L of KI, all adjusted to pH 7.0. The fermenter was seeded with 3 L of vegetative inoculum and incubated for 120-192 hours at 37°C, with aeration of 0.5 volume/volume/minute (vvm), relative pressure of 0.5 barg and stirring at 150 rpm. The obtained results are shown in Figure 6.

### Example 8. Production of ADD in a fermenter with the Mycobacterium smegmatis CECT 8331 (MS6039) strain using phytosterol and vegetable oil

Fermentation was performed in 2 phases using a vegetative inoculum and fermenter. The vegetative inoculum was seeded with 1 ml of microorganism (about 10⁸ cells) and incubated in a 5 L fermenter with 3 L of culture medium with the following composition: 54 g/L of molasses, 5.4 g/L of NaNo₃, 0.6 g/L of NH₄H₂PO₄, 20 g/L of soybean oil and 20 µg/ml of kanamycin, all adjusted to pH 7.0. The vegetative inoculum was incubated for 48-72 hours at 37°C, with aeration of 0.5 volume/volume/minute (vvm), relative pressure of 0.5 barg and stirring at 150 rpm. Cell pellet volumes of 5-20% analyzed by centrifugation for 10 minutes at 2,000 x *g* were thereby obtained.

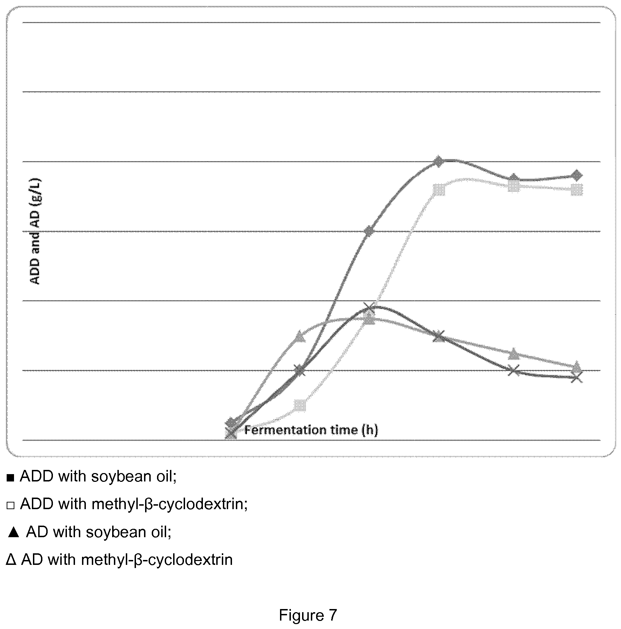

Fermentation (biotransformation) was performed in a 42 L fermenter with 30 L of culture medium with the following composition (g/L): 2 g/L of NH₄NO₃, 1 g/L of KH₂PO₄, 2 g/L of K₂HPO₄, 0.2 g/L of KCI, 0.3 g/L of CaCl₂, 150 g/L of soybean oil, 20 µg/ml of kanamycin, and 20-40 g/L of phytosterol; 1 mL of a mixture of micronutrients was additionally added for each L of fermentation medium. The composition of the mixture of micronutrients is as follows: 11 g/L of ZnSO₄, 1 g/L of FeSO₄, 6 g/L of MnSO₄, 0.04 g/L of CuSO₄, 0.3 g/L of CoSO₄, 0.03 g/L of H₃PO₃, and 0.001 g/L of KI, all adjusted to pH 7.0. The fermenter was seeded with 3 L of vegetative inoculum and incubated for 120-192 hours at 37°C, with aeration of 0.5 volume/volume/minute (vvm), relative pressure of 0.5 barg and stirring at 150 rpm. The obtained results are shown in Figure 7.

### Example 9. Production of ADD in a fermenter with the Mycobacterium smegmatis CECT 8331 (MS6039) strain using phytosterol and methyl-β-cyclodextrin

Fermentation was performed in 2 phases using a vegetative inoculum and fermenter. The vegetative inoculum was seeded with 1 ml of microorganism (about 10⁸ cells) and incubated in a 5 L fermenter with 3 L of culture medium with the following composition: 54 g/L of molasses, 5.4 g/L of NaNo₃, 0.6 g/L of NH₄H₂PO₄, 20 g/L of soybean oil, and 20 µg/ml of kanamycin, all adjusted to pH 7.0. The vegetative inoculum was incubated for 48-72 hours at 37°C, with aeration of 0.5 volume/volume/minute (vvm), relative pressure of 0.5 barg and stirring at 150 rpm. Cell pellet volumes of 5-20% analyzed by centrifugation for 10 minutes at 2,000 x *g* were thereby obtained.

Fermentation (biotransformation) was performed in a 42 L fermenter with 30 L of culture medium with the following composition (g/L): 2 g/L of NH₄NO₃, 1 g/L of KH₂PO₄, 2 g/L of K₂HPO₄, 0.2 g/L of KCI, 0.3 g/L of CaCl₂, 40 g/L of methyl-β-cyclodextrin (Cavasol® W7M, Wacker Chemie), 20 µg/ml of kanamycin, and 20 g/L of phytosterol; 1 mL of a mixture of micronutrients was additionally added for each L of fermentation medium. The composition of the mixture of micronutrients is as follows: 11 g/L of ZnSO₄, 1 g/L of FeSO₄, 6 g/L of MnSO₄, 0.04 g/L of CuSO₄, 0.3 g/L of CoSO₄, 0.03 g/L of H₃PO₃, and 0.001 g/L of KI, all adjusted to pH 7.0. The fermenter was seeded with 3 L of vegetative inoculum and incubated for 120-192 hours at 37°C, with aeration of 0.5 volume/volume/minute (vvm), relative pressure of 0.5 barg and stirring at 150 rpm. The obtained results are shown in Figure 7.

### SEQUENCE LISTING FOR SEQ ID NO 1 AND SEQ ID NO 2

SEQ ID NO 1 (MSMEG_6039)
SEQ ID NO 2 (MSMEG_5941)

## Claims

1. A genetically modified strain of the species *Mycobacterium smegmatis (M. smegmatis),* **characterized in that** *said strain is uncapable of using any of substances AD* (4-androstene-3,17-dione) *or ADD* (1,4-androstadiene-3,17-dione) *as a carbon source when said substances are added to a culture medium,* and **in that** in said strain a reductase component of 3-ketosteroid 9 α-hydroxylase enzyme has been functionally inactivated or completely or partially deleted, wherein at least one nucleotide sequence encoding the reductase component of 3-ketosteroid 9 α-hydroxylase enzyme corresponds with: a) the sequence having SEQ ID NO 1; or b) a variant to the sequence having SEQ ID NO 1 which is at least 75% homologous to SEQ ID NO 1, based on the identity of all the nucleotides of said sequence, and wherein said strain is capable of producing ADD with a purity of 95% or more with respect to the AD content by fermentation in a minimal culture medium containing cholesterol or a mixture of phytosterols and glycerol as an alternative carbon source to cholesterol.

2. The genetically modified strain of claim 1, wherein the parent strain that is genetically modified to give the genetically modified strain of claim 1 is M. smegmatis mc²155 bacterium.

3. The strain according to claim 1 or 2, **characterized in that** the nucleotide sequence encoding the reductase component of 3-ketosteroid 9α-hydroxylase enzyme corresponds with the sequence having SEQ ID NO 1.

4. The strain according to any one of claims 1 to 3, **characterized in that** the nucleotide sequence encoding the reductase component of 3-ketosteroid 9α-hydroxylase enzyme is completely or partially deleted.

5. The strain according to any one of claims 1 to 4, **characterized in that** it corresponds to the strain deposited in the Spanish Type Culture Collection with accession number CECT 8331 with depositor identification reference number MS6039 on July 4, 2013.

6. A genetically modified strain of the species *M. smegmatis,* **characterized in that** *said strain is uncapable of using any of substances AD or ADD as a carbon source when said substances are added to a culture medium,* and **in that** in said strain at least one nuleotide sequence encoding a reductase component of 3-ketosteroid 9 α-hydroxylase enzyme has been functionally inactivated or completely or partially deleted, and at least one nucleotide sequence encoding 3-ketosteroid-Δ1-dehydrogenase enzyme has been functionally inactivated or completely or partially deleted, wherein the nucleotide sequence encoding the reductase component of 3-ketosteroid 9 α-hydroxylase enzyme corresponds with: a) the sequence having SEQ ID NO 1; or b) a variant to the sequence having SEQ ID NO 1 which is at least 75% homologous to SEQ ID NO 1, based on the identity of all the nucleotides of said sequence, and wherein the nucleotide sequence encoding 3-ketosteroid-Δ1-dehydrogenase enzyme corresponds with: a) the sequence having SEQ ID NO 2; or b) a variant to the sequence having SEQ ID NO 2 which is at least 75% homologous to SEQ ID NO 2, based on the identity of all the nucleotides of said sequence, and wherein said genetically modified strain is capable of producing AD with a purity of 95% or more with respect to the ADD content by fermentation in a minimal culture medium containing cholesterol or a mixture of phytosterols and glycerol as an alternative carbon source to cholesterol.

7. The strain according to claim 6, **characterized in that** the nucleotide sequence encoding the reductase component of 3-ketosteroid 9 α-hydroxylase enzyme corresponds with the sequence having SEQ ID NO 1.

8. The strain according to any one of claims 6 to 7, **characterized in that** the nucleotide sequence encoding 3-ketosteroid-Δ1-dehydrogenase enzyme corresponds with the sequence having SEQ ID NO 2.

9. The strain according to any one of claims 6 to 8, **characterized in that** it corresponds to the strain deposited in the Spanish Type Culture Collection with accession number CECT 8332 with depositor identification reference MS6039-5941 on July 4, 2013.

10. Use of the strain defined in any of claims 1 to 5 for producing ADD with a purity of 95% or more with respect to the AD content by means of fermenting natural sterols in a minimal culture medium containing cholesterol or a mixture of phytosterols and an alternative carbon source to cholesterol such as glycerol.

11. Use of the strain defined in any of claims 6 to 9 for producing AD with a purity of 95% or more with respect to the ADD content by means of fermenting natural sterols in a minimal culture medium containing cholesterol or a mixture of phytosterols and an alternative carbon source to cholesterol such as glycerol.

12. Use according to any one of claims 10 or 11, **characterized in that** the natural sterols are selected from cholesterol or phytosterols.

13. A method for obtaining ADD with a purity of 95% or more with respect to the AD content in a minimal culture medium containing cholesterol or a mixture of phytosterols and an alternative carbon source to cholesterol such as glycerol, **characterized in that** it comprises the steps of:
a. preparing and sterilizing a mixture of a natural sterol or a mixture of natural sterols in a polyalcohol or a vegetable oil, with the addition of mineral salts;
b. growing a culture of the strain defined in any one of claims 1 to 5 in the mixture obtained in a);
c. separating the cell pellet to obtain a solution containing ADD.

14. A method for obtaining AD with a purity of 95% or more with respect to the ADD content in a minimal culture medium containing cholesterol or a mixture of phytosterols and an alternative carbon source to cholesterol such as glycerol, **characterized in that** it comprises the steps of:
a. preparing and sterilizing a mixture of a natural sterol or a mixture of natural sterols in a polyalcohol or a vegetable oil, with the addition of mineral salts;
b. growing a culture of the strain defined in any one of claims 6 to 9 in the mixture obtained in a);
c. separating the cell pellet to obtain a solution containing AD.

15. A method for obtaining ADD with a purity of 95% or more with respect to the AD content in a minimal culture medium containing cholesterol or a mixture of phytosterols and an alternative carbon source to cholesterol such as glycerol, **characterized in that** it comprises the steps of:
a. preparing a mixture of a natural sterol or a mixture of natural sterols in a polyalcohol or a vegetable oil;
b. preparing a culture of the strain defined in any one of claims 1 to 5;
c. incubating the mixture from a) with the cell culture from b);
d. separating the cell pellet to obtain a solution containing ADD.

16. A method for obtaining AD with a purity of 95% or more with respect to the ADD content in a minimal culture medium containing cholesterol or a mixture of phytosterols and an alternative carbon source to cholesterol such as glycerol, **characterized in that** it comprises the steps of:
a. preparing a mixture of a natural sterol or a mixture of natural sterols in a polyalcohol or a vegetable oil;
b. preparing a culture of the strain defined in any one of claims 6 to 9;
c. incubating the mixture from a) with the cell culture from b);
d. separating the cell pellet to obtain a solution containing AD.

## Patentansprüche

1. Genetisch veränderter Stamm der Art *Mycobacterium smegmatis* (*M. smegmatis*), **dadurch gekennzeichnet, dass** der genannte Stamm nicht in der Lage ist, jede der Substanzen AD (4-Androsten-3,17-dion) oder ADD (1,4-Androstadien-3,17-dion) als Kohlenstoffquelle zu verwenden, wenn die genannten Substanzen einem Kulturmedium hinzugefügt werden, und dass im genannten Stamm eine Reduktase-Komponente des Enzyms 3-Ketosteroid 9α-Hydroxylase funktionell inaktiviert oder vollständig oder teilweise deletiert worden ist, wobei mindestens eine Nukleotidsequenz, welche für die Reduktase-Komponente des Enzyms 3-Ketosteroid 9 α-Hydroxylase kodiert, Folgendem entspricht: a) der Sequenz aufweisend SEQ ID NO 1; oder b) einer Variante der Sequenz aufweisend SEQ ID NO 1, welche mindestens 75% homolog zu SEQ ID NO 1 ist, basierend auf der Identität der gesamten Nukleotide der genannten Sequenz, und wobei der genannte Stamm in der Lage ist, ADD mit einer Reinheit von 95% oder mehr in Bezug auf den AD-Gehalt mittels Fermentation in einem minimalen Kulturmedium beinhaltend Cholesterin oder eine Mischung aus Phytosterinen und Glycerin als alternative Kohlenstoffquelle zu Cholesterin herzustellen.

2. Genetisch veränderter Stamm nach Anspruch 1, wobei der Ausgangsstamm, welcher genetisch verändert wird, um den genetisch veränderten Stamm nach Anspruch 1 bereitzustellen, die Bakterie *M. smegmatis* mc² 155 ist.

3. Stamm nach Anspruch 1 oder 2, **dadurch gekennzeichnet, dass** die Nukleotidsequenz, welche für die Reduktase-Komponente des Enzyms 3-Ketosteroid 9 α-Hydroxylase kodiert, der Sequenz aufweisend SEQ ID NO 1 entspricht.

4. Stamm nach einem der Ansprüche 1 bis 3, **dadurch gekennzeichnet, dass** die Nukleotidsequenz, welche für die Reduktase-Komponente des Enzyms 3-Ketosteroid 9 α-Hydroxylase kodiert, vollständig oder teilweise deletiert ist.

5. Stamm nach einem der Ansprüche 1 bis 4, **dadurch gekennzeichnet, dass** er dem Stamm entspricht, welcher in der Spanischen Kultursammlung mit der Zugangsnummer CECT 8331 mit der Hinterleger-Identifikations-/Referenznummer MS6039 am 4. Juli 2013 hinterlegt wurde.

6. Genetisch veränderter Stamm der Art *M. smegmatis,* **dadurch gekennzeichnet, dass** der genannte Stamm nicht in der Lage ist, jede der Substanzen AD oder ADD als Kohlenstoffquelle zu verwenden, wenn die genannten Substanzen einem Kulturmedium hinzugefügt werden, und dass im genannten Stamm mindestens eine Nukleotidsequenz, welcher für eine Reduktase-Komponente des Enzyms 3-Ketosteroid 9 α-Hydroxylase kodiert, funktionell inaktiviert oder vollständig oder teilweise deletiert worden ist, und mindestens eine Nukleotidsequenz, welche für das Enzym 3-Ketosteroid-Δ1-Dehydrogenase kodiert, funktionell inaktiviert oder vollständig oder teilweise deletiert worden ist, wobei die Nukleotidsequenz, welche für die Reduktase-Komponente des Enzyms 3-Ketosteroid 9 α-Hydroxylase kodiert, Folgendem entspricht: a) der Sequenz aufweisend SEQ ID NO 1; oder b) einer Variante der Sequenz aufweisend SEQ ID NO 1, welche mindestens 75% homolog zu SEQ ID NO 1 ist, basierend auf der Identität der gesamten Nukleotide der genannten Sequenz, und wobei die Nukleotidsequenz, welche für das Enzym 3-Ketosteroid-Δ1-Dehydrogenase kodiert, Folgendem entspricht: a) der Sequenz aufweisend SEQ ID NO 2; oder b) einer Variante der Sequenz aufweisend SEQ ID NO 2, welche mindestens 75% homolog zu SEQ ID NO 2 ist, basierend auf der Identität der gesamten Nukleotide der genannten Sequenz, und wobei der genannte genetisch veränderte Stamm in der Lage ist, AD mit einer Reinheit von 95% oder mehr in Bezug auf den ADD-Gehalt mittels Fermentation in einem minimalen Kulturmedium beinhaltend Cholesterin oder eine Mischung aus Phytosterinen und Glycerin als alternative Kohlenstoffquelle zu Cholesterin herzustellen.

7. Stamm nach Anspruch 6, **dadurch gekennzeichnet, dass** die Nukleotidsequenz, welche für die Reduktase-Komponente des Enzyms 3-Ketosteroid 9 α-Hydroxylase kodiert, der Sequenz aufweisend SEQ ID NO 1 entspricht.

8. Stamm nach einem der Ansprüche 6 bis 7, **dadurch gekennzeichnet, dass** die Nukleotidsequenz, welche für das Enzym 3-Ketosteroid-Δ1-Dehydrogenase kodiert, der Sequenz aufweisend SEQ ID NO 2 entspricht.

9. Stamm nach einem der Ansprüche 6 bis 8, **dadurch gekennzeichnet, dass** er dem Stamm entspricht, welcher in der Spanischen Kultursammlung mit der Zugangsnummer CECT 8332 mit der Hinterleger-Identifikation/Referenz MS6039-5941 am 4. Juli 2013 hinterlegt wurde.

10. Verwendung des in einem der Ansprüche 1 bis 5 definierten Stammes zur Herstellung von ADD mit einer Reinheit von 95% oder mehr in Bezug auf den AD-Gehalt mittels Fermentation von natürlichen Sterolen in einem minimalen Kulturmedium beinhaltend Cholesterin oder eine Mischung aus Phytosterinen und eine alternative Kohlenstoffquelle zu Cholesterin, wie Glycerin.

11. Verwendung des in einem der Ansprüche 6 bis 9 definierten Stammes zur Herstellung von AD mit einer Reinheit von 95% oder mehr in Bezug auf den ADD-Gehalt mittels Fermentation von natürlichen Sterolen in einem minimalen Kulturmedium beinhaltend Cholesterin oder eine Mischung aus Phytosterinen und eine alternative Kohlenstoffquelle zu Cholesterin, wie Glycerin.

12. Verwendung nach einem der Ansprüche 10 oder 11, **dadurch gekennzeichnet, dass** die natürlichen Sterole aus Cholesterin oder Phytosterinen ausgewählt werden.

13. Verfahren zum Erhalten von ADD mit einer Reinheit von 95% oder mehr in Bezug auf den AD-Gehalt in einem minimalen Kulturmedium beinhaltend Cholesterin oder eine Mischung aus Phytosterinen und eine alternative Kohlenstoffquelle zu Cholesterin, wie Glycerin, **dadurch gekennzeichnet, dass** es die folgenden Schritte umfasst:
a. das Zubereiten und Sterilisieren einer Mischung aus einem natürlichen Sterol oder einer Mischung aus natürlichen Sterolen in einem Polyalkohol oder einem pflanzlichen Öl, mit der Hinzufügung von Mineralsalzen;
b. das Wachsenlassen einer Kultur des in einem der Ansprüche 1 bis 5 definierten Stammes in der in a) erhaltenen Mischung;
c. das Trennen des Zellenpellets, um eine Lösung beinhaltend ADD zu erhalten.

14. Verfahren zum Erhalten von AD mit einer Reinheit von 95% oder mehr in Bezug auf den ADD-Gehalt in einem minimalen Kulturmedium beinhaltend Cholesterin oder eine Mischung aus Phytosterinen und eine alternative Kohlenstoffquelle zu Cholesterin, wie Glycerin, **dadurch gekennzeichnet, dass** es die folgenden Schritte umfasst:
a. das Zubereiten und Sterilisieren einer Mischung aus einem natürlichen Sterol oder einer Mischung aus natürlichen Sterolen in einem Polyalkohol oder einem pflanzlichen Öl, mit der Hinzufügung von Mineralsalzen;
b. das Wachsenlassen einer Kultur des in einem der Ansprüche 6 bis 9 definierten Stammes in der in a) erhaltenen Mischung;
c. das Trennen des Zellenpellets, um eine Lösung beinhaltend AD zu erhalten.

15. Verfahren zum Erhalten von ADD mit einer Reinheit von 95% oder mehr in Bezug auf den AD-Gehalt in einem minimalen Kulturmedium beinhaltend Cholesterin oder eine Mischung aus Phytosterinen und eine alternative Kohlenstoffquelle zu Cholesterin, wie Glycerin, **dadurch gekennzeichnet, dass** es die folgenden Schritte umfasst:
a. das Zubereiten einer Mischung aus einem natürlichen Sterol oder einer Mischung aus natürlichen Sterolen in einem Polyalkohol oder einem pflanzlichen Öl;
b. das Zubereiten einer Kultur des in einem der Ansprüche 1 bis 5 definierten Stammes;
c. das Inkubierten der Mischung aus a) mit der Zellkultur aus b);
d. das Trennen des Zellenpellets, um eine Lösung beinhaltend ADD zu erhalten.

16. Verfahren zum Erhalten von AD mit einer Reinheit von 95% oder mehr in Bezug auf den ADD-Gehalt in einem minimalen Kulturmedium beinhaltend Cholesterin oder eine Mischung aus Phytosterinen und eine alternative Kohlenstoffquelle zu Cholesterin, wie Glycerin, **dadurch gekennzeichnet, dass** es die folgenden Schritte umfasst:
a. das Zubereiten einer Mischung aus einem natürlichen Sterol oder einer Mischung aus natürlichen Sterolen in einem Polyalkohol oder einem pflanzlichen Öl;
b. das Zubereiten einer Kultur des in einem der Ansprüche 6 bis 9 definierten Stammes;
c. das Inkubierten der Mischung aus a) mit der Zellkultur aus b);
d. das Trennen des Zellenpellets, um eine Lösung beinhaltend AD zu erhalten.

## Revendications

1. Souche génétiquement modifiée de l'espèce *Mycobacterium smegmatis* (*M. smegmatis*), **caractérisée en ce que** ladite souche est incapable d'utiliser aucune des substances AD (4-androstène-3,17-dione) ou ADD (1,4-androstadiène-3,17-dione) en tant que source de carbone lorsque lesdites substances sont ajoutées à un milieu de culture, et **en ce que** dans ladite souche un composant de réductase de l'enzyme 3-cétostéroïde 9-α-hydroxylase a été inactivée fonctionnellement ou délétée complètement ou partiellement, dans laquelle au moins une séquence nucléotidique codant le composant de réductase de l'enzyme 3-cétostéroïde 9-α-hydroxylase correspond à : a) la séquence ayant SEQ ID NO 1; ou b) une variante à la séquence ayant SEQ ID NO 1 qui est au moins 75% homologue à SEQ ID NO 1, basée sur l'identité de tous les nucléotides de ladite séquence, et dans laquelle ladite souche est capable de produire ADD avec une pureté de 95% ou plus par rapport au contenu d'AD par fermentation dans un milieu de culture minimal contenant du cholestérol ou un mélange de phytostérols et de glycérol en tant que source de carbone alternative au cholestérol.

2. Souche génétiquement modifiée selon la revendication 1, dans laquelle la souche parentale qui est modifiée génétiquement pour fournir la souche génétiquement modifiée de la revendication 1 est la bactérie *M*. *smegmatis* mc² 155.

3. Souche selon la revendication 1 ou 2, **caractérisée en ce que** la séquence nucléotidique codant le composant de réductase de l'enzyme 3-cétostéroïde 9-α-hydroxylase correspond à la séquence ayant SEQ ID NO 1.

4. Souche selon l'un quelconque des revendications 1 à 3, **caractérisée en ce que** la séquence nucléotidique codant le composant de réductase de l'enzyme 3-cétostéroïde 9-α-hydroxylase est délétée complètement ou partiellement.

5. Souche selon l'un quelconque des revendications 1 à 4, **caractérisée en ce qu'**elle correspond à la souche déposée dans la Collection espagnole de cultures type avec le numéro d'accession CECT 8331 avec le numéro de référence d'identification du déposant MS6039 le 4 juillet 2013.

6. Souche génétiquement modifiée de l'espèce *M. smegmatis,* **caractérisée en ce que** ladite souche est incapable d'utiliser aucune des substances AD ou ADD en tant que source de carbone lorsque lesdites substances sont ajoutées à un milieu de culture, et **en ce que** dans ladite souche au moins une séquence nucléotidique codant un composant de réductase de l'enzyme 3-cétostéroïde 9-α-hydroxylase a été inactivée fonctionnellement ou délétée complètement ou partiellement, et au moins une séquence nucléotidique codant l'enzyme 3-cétostéroïde-Δ1-déhydrogénase a été fonctionnellement inactivée ou délétée complètement ou partiellement, dans laquelle la séquence nucléotidique codant le composant de réductase de l'enzyme 3-cétostérïde 9α-hydroxylase correspond à a) la séquence ayant SEQ ID NO 1 ; ou b) une variante à la séquence ayant SEQ ID NO 1 qui est au moins 75% homologue à SEQ ID NO 1, basée sur l'identité de tous les nucléotides de ladite séquence, et dans laquelle ladite séquence nucléotidique codant l'enzyme 3-cétostéroïde-Δ1-déhydrogénase correspond à : a) la séquence ayant SEQ ID NO 2 ; ou b) une variante à la séquence ayant SEQ ID NO 2 qui est au moins 75% homologue à SEQ ID NO 2, basée sur l'identité de tous les nucléotides de ladite séquence, et dans laquelle ladite souche génétiquement modifiée est capable de produire AD avec une pureté de 95% ou plus par rapport au contenu d'ADD par fermentation dans un milieu de culture minimal contenant du cholestérol ou un mélange de phytostérols et de glycérol en tant que source de carbone alternative au cholestérol.

7. Souche selon la revendication 6, **caractérisée en ce que** la séquence nucléotidique codant le composant de réductase de l'enzyme 3-cétostéroïde 9-α-hydroxylase correspond à la séquence ayant SEQ ID NO 1.

8. Souche selon l'un quelconque des revendications 6 à 7, **caractérisée en ce que** la séquence nucléotidique codant l'enzyme 3-cétostéroïde-Δ1-déhydrogénase correspond à la séquence ayant SEQ ID NO 2.

9. Souche selon l'un quelconque des revendications 6 à 8, **caractérisée en ce qu'**elle correspond à la souche déposée dans la Collection espagnole de cultures type avec le numéro d'accession CECT 8332 avec le numéro de référence d'identification du déposant MS6039-5941 le 4 juillet 2013.

10. Utilisation de la souche définie dans l'une quelconque des revendications 1 à 5 pour produire ADD avec une pureté de 95% ou plus par rapport au contenu d'AD par le biais de la fermentation de stérols naturels dans un milieu de culture minimal contenant du cholestérol ou un mélange de phytostérols et une source de carbone alternative au cholestérol comme le glycérol.

11. Utilisation de la souche définie dans l'une quelconque des revendications 6 à 9 pour produire AD avec une pureté de 95% ou plus par rapport au contenu d'ADD par le biais de la fermentation de stérols naturels dans un milieu de culture minimal contenant du cholestérol ou un mélange de phytostérols et une source de carbone alternative au cholestérol comme le glycérol.

12. Utilisation selon l'une quelconque des revendications 10 ou 11, **caractérisée en ce que** les stérols naturels sont choisis parmi le cholestérol ou les phytostérols.

13. Procédé d'obtention d'ADD avec une pureté de 95% ou plus par rapport au contenu d'AD dans un milieu de culture minimal contenant du cholestérol ou un mélange de phytostérols et une source de carbone alternative au cholestérol comme le glycérol, **caractérisé en ce qu'**il comprend les étapes de :
a. préparer et stériliser un mélange d'un stérol naturel ou un mélange de stérols naturels dans un polyalcool ou une huile végétale, avec l'addition de sels minéraux ;
b. développer une culture de la souche définie dans l'une quelconque des revendications 1 à 5 dans le mélange obtenu dans a) ;
c. séparer le culot de cellules pour obtenir une solution contenant ADD.

14. Procédé d'obtention d'AD avec une pureté de 95% ou plus par rapport au contenu d'ADD dans un milieu de culture minimal contenant du cholestérol ou un mélange de phytostérols et une source de carbone alternative au cholestérol comme le glycérol, **caractérisé en ce qu'**il comprend les étapes de :
a. préparer et stériliser un mélange d'un stérol naturel ou un mélange de stérols naturels dans un polyalcool ou une huile végétale, avec l'addition de sels minéraux ;
b. développer une culture de la souche définie dans l'une quelconque des revendications 6 à 9 dans le mélange obtenu dans a) ;
c. séparer le culot de cellules pour obtenir une solution contenant AD.

15. Procédé d'obtention d'ADD avec une pureté de 95% ou plus par rapport au contenu d'AD dans un milieu de culture minimal contenant du cholestérol ou un mélange de phytostérols et une source de carbone alternative au cholestérol comme le glycérol, **caractérisé en ce qu'**il comprend les étapes de :
a. préparer un mélange d'un stérol naturel ou un mélange de stérols naturels dans un polyalcool ou une huile végétale;
b. préparer une culture de la souche définie dans l'une quelconque des revendications 1 à 5 ;
c. incuber le mélange de a) avec la culture cellulaire de b) ;
d. séparer le culot de cellules pour obtenir une solution contenant ADD.

16. Procédé d'obtention d'AD avec une pureté de 95% ou plus par rapport au contenu d'ADD dans un milieu de culture minimal contenant du cholestérol ou un mélange de phytostérols et une source de carbone alternative au cholestérol comme le glycérol, **caractérisé en ce qu'**il comprend les étapes de :
a. préparer un mélange d'un stérol naturel ou un mélange de stérols naturels dans un polyalcool ou une huile végétale ;
b. préparer une culture de la souche définie dans l'une quelconque des revendications 6 à 9 ;
c. incuber le mélange de a) avec la culture cellulaire de b) ;
d. séparer le culot de cellules pour obtenir une solution contenant AD.
